# EUROPEAN PATENT SPECIFICATION

(11) **EP 2 444 062 B1**
(45) Date of publication and mention of the grant of the patent: **10.05.2017**
(21) Application number: 11168833.9
(22) Date of filing: 06.06.2011
(51) Int. Cl.: A61K 8/81, A61Q 1/02, A61Q 1/04, A61Q 1/06, A61Q 1/10, A61Q 3/02, A61Q 5/02, A61Q 15/00, A61Q 17/04, A61Q 19/00, A61Q 19/10, C08F 132/08, A61K 8/891, A61Q 1/08, A61Q 3/00, A61Q 5/00, A61Q 1/12, A61Q 19/08, C08F 230/08, C08F 232/08

(54) **Cosmetic product containing film-forming polymer**
Filmbildner-Polymer mit kosmetischem Produkt
Produit cosmétique contenant un polymère filmogène

(30) Priority: 08.06.2010 JP 2010130784
(43) Date of publication of application: 25.04.2012
(73) Proprietor: Shin-Etsu Chemical Co., Ltd., Chiyoda-ku, Tokyo (JP)
(72) Inventor: Hayakawa, Chihiro, Tokyo, Gunma (JP); Tetsuka, Hiroaki, Annaka-shi, Gunma (JP); Yoneda, Yoshinori, Annaka-shi, Gunma (JP); Hagiwara, Mamoru, Annaka-shi, Gunma (JP)
(74) Representative: Stoner, Gerard Patrick

(56) References cited:
- EP-A1- 2 213 691
- EP-A1- 2 412 732
- WO-A2-2008/087325
- GB-A- 2 407 496
- JP-A- 2002 322 042
- JP-A- 2009 173 824
- JP-A- 2009 249 610
- JP-A- 2010 024 154
- JP-A- 2011 084 681
- US-A1- 2010 326 121
- TETSUKA H ET AL: "Synthesis and properties of addition-type poly(norbornene)s with siloxane substituent", POLYMER JOURNAL, SOCIETY OF POLYMER SCIENCE, TOKYO, JP, vol. 41, no. 8, 1 January 2009 (2009-01-01), pages 643-649, XP009163984, ISSN: 0032-3896
- TETSUKA ET AL: "Addition-type poly(norbornene)s with siloxane substituents : synthesis, properties and nanoporous membrane", POLYMER JOURNAL, [Online] vol. 43, 20 October 2010 (2010-10-20), pages 97-100, XP002685840,

## Description

### TECHNICAL FIELD

The present invention relates to a cosmetic product and, more particularly, to the one containing a film-forming polymer composed of building blocks with cycloolefin skeletons. Said polymer has good compatibility with a variety of oils, especially silicone oil, readily spreads on the skin and hair without sticky feeling, and prevents the cosmetic product from staining clothes.

### BACKGROUND

Cosmetic products are usually incorporated with a film-forming polymer so that they keep long. There is much need for development of make-up cosmetic products and sun care cosmetic product superior in water resistance and perspiration resistance. This need has been met by incorporation with trimethylsiloxy silicic acid or silicone-modified acrylic resin. However, when added sufficiently, the former gives a squeaking feel after application on account of a hard film it produces. It also produces a brittle film which easily peels off when pressed or stretched. Moreover, cosmetic products containing it easily stick to clothes when scratched by clothes after application. (This phenomenon will be referred to as "secondary sticking" hereinafter.) By contrast, said silicone-modified acrylic resin gives a soft good feel but has a bit of problem in secondary sticking due to its weak film.

Another known additive is a polycycloolefin polymer (as disclosed in JP-A 2007-291150). This polymer is superior in film-forming performance and finds use as gas-permeable film and contact lenses.

For more information, refer to JP-A 2010-24154 which describes ROMP - synthesised polycycloolefins with siloxane substituents used in cosmetics, and JP-A-2002/322042 describing the use of polynorbornenes in skin care preparations.

The present inventors found that such problems with film-forming polymers in the state of the art can be addressed or solved using a polycycloolefin polymer whose skeleton has specific functional groups. This led to the present invention.

The present invention, therefore, provides the following cosmetic products containing film-forming polymer.
[1] A cosmetic product comprising a polymer having the repeating unit represented by the following formula (1) or (2): 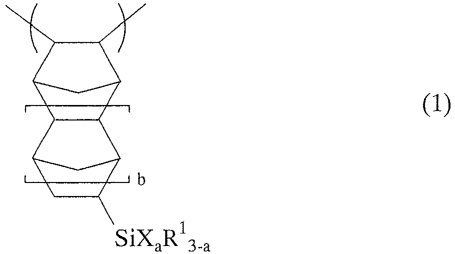 wherein R¹ is independently alkyl group having 1 to 12 carbon atoms, X is a group represented by the following formula (i), a is an integer of 1 to 3, and b is an integer of 0 to 2, 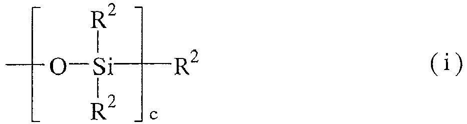 wherein R² is independently alkyl group having 1 to 12 carbon atoms and c is an integer of 1 to 5, 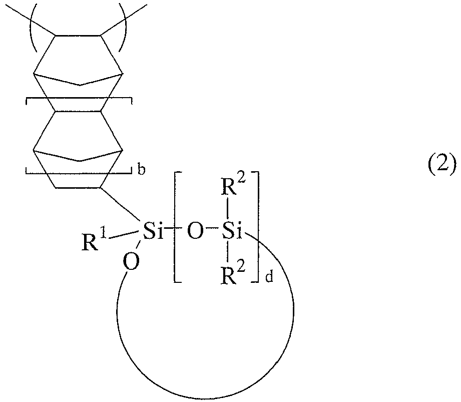 wherein R¹, R², and b are defined as above, and d is an integer of 2 to 5; and additionally
   (C) compound which contains alcoholic hydroxyl group in its molecular structure, as set out in claim 1.
[2] The cosmetic product according to [1] above wherein said polymer further has the repeating unit represented by the following formula (3): 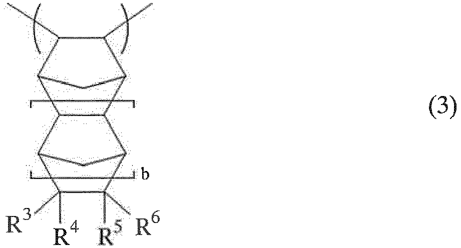 wherein R³ to R⁶ are independently selected from the group consisting of
   hydrogen atoms, halogen atoms,
   substituents selected from C1-10 alkyl groups, alkenyl groups, cycloalkyl groups, aryl groups, alkoxy groups, aryloxy groups and halogenated hydrocarbon groups,
   polar groups selected from oxetanyl groups, alkoxycarbonyl groups, polyoxyalkylene groups and polyglyceryl groups, and
   alkoxysilyl groups. Any two of R³ to R⁶ may form, in conjunction with carbon atoms to which they join, an alicyclic structure, aromatic ring structure, carbonimide group, or acid anhydride. b is an integer of 0 to 2.
[3] The cosmetic product according to [1] or [2] above, wherein said polymer has a number-average molecular weight (Mn) of 10,000 to 2,000,000 in terms of polystyrene determined by Gel Permeation Chromatography.
[4] The cosmetic product according to [1], [2] or [3] above, further comprising a volatile silicone oil.
[5] The cosmetic product according to [4] above, wherein said volatile silicone oil is one selected from the group consisting of octamethyltrisiloxane, decamethyltetrasiloxane, octamethylcyclotetrasiloxane, decamethylcyclopentasiloxane, tristrimethylsiloxymethylsilane, tetrakistrimethylsiloxysilane and dodecamethylpentasiloxane.
[6] The cosmetic product according to [1], further comprising isododecane.

Use of the above product as a cosmetic product is another aspect herein.

Addition-type norbornene polymers and copolymers of the above types were disclosed as such, and in the forms of films, sheets or membranes, in Polymer Journal, Vol. 41, No. 8, pp 643-649, 2009 "Synthesis and Properties of Addition-Type Poly(norbornene)s with Siloxane Substituents", H. Tetsuka et al, and in JP-A-2009/249610 and JP-A-2009/173824.

### ADVANTAGEOUS EFFECTS

The cosmetic products proposed herein contain a polymer capable of forming a coating film which is soft, tough and water resistant. We find that they avoid or prevent secondary sticking, keep a long time without peeling, and impart a good feeling.

### FURTHER EXPLANATIONS; OPTIONS AND PREFERENCES

About the present invention, a description will hereinafter be made in the order of (I) polymer, (II) Making method of polymer, and (III) cosmetic product.

### (I) Polymer

The polymer used in the cosmetic product according to the present invention is one which has the repeating unit represented by the formula (1) or (2). The one represented by the formula (1) has diorganopolysiloxane residues of chain structure, and the one represented by the formula (2) has diorganopolysiloxane residues of cyclic structure. The residues in the former should preferably be those of branched chain structure. The polymer includes copolymers, and it may have the repeating units represented by the formulas (1) and (2) in combination with one another. The copolymers may be block copolymers or random copolymers. 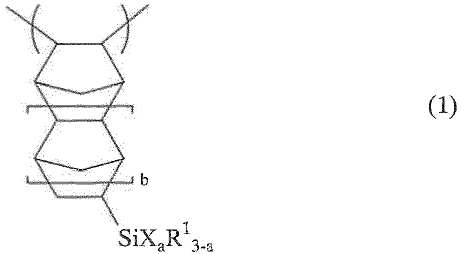 wherein R¹ is independently alkyl group having 1 to 12 carbon atoms, X is a group represented by the following formula (i), a is an integer of 1 to 3, and b is an integer of 0 to 2, 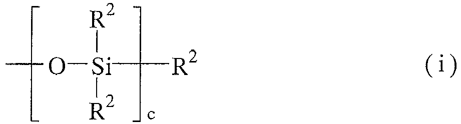 wherein R² is independently alkyl group having 1 to 12 carbon atoms and c is an integer of 1 to 5, 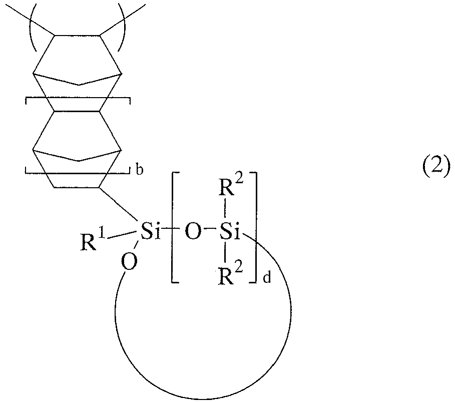 wherein R¹, R², and b are defined as above, and d is an integer of 2 to 5.

In the formula (1) above, R¹ is independently alkyl group having 1 to 12 carbon atoms, such as methyl group, ethyl group, n-propyl group, butyl group, and pentyl group. Preferable among them is methyl group.
a is an integer of 1 to 3. The polymer may have two repeating units (a = 2) and three repeating units (a = 3) in combination. The one in which a is 3 is preferable from the standpoint of polymerizability. b is an integer of 0 to 2. In the polymer, b may be 0 or 1 or a combination thereof, and should preferably be 0.
X is a group represented by the formula (i) above. In the formula (i), R² is independently alkyl group having 1 to 12 carbon atoms, which include those groups defined by R¹ above. A methyl group is preferable, c is an integer of 1 to 5, and the preferred value of c is 1.

The group represented by the formula (i) may be exemplified by the trimethylsiloxy group shown as following.

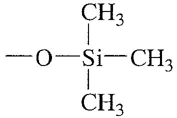

In the formula (2) above, R¹, R², and b are defined as above, and d is an integer of 2 to 5, with 2 being preferable. The diorganopolysiloxane residue of cyclic structure shown in the formula (2) should preferably be one in which R² is a methyl group and d is 2 or 3 as represented by the following formula (4) (d = 2) or (5) (d = 3), respectively.

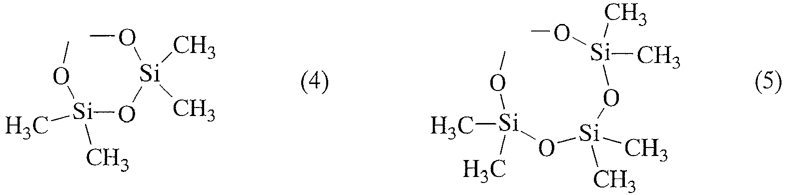

The rate of the repeating units (1) and (2) is preferably 40 to 95%, more preferably 60 to 90%, of all the repeating units.

The copolymer may contain, in addition to the repeating units (1) and (2), the repeating unit (3) represented by the following formula (3). The repeating units (1), (2), and (3) may join randomly. 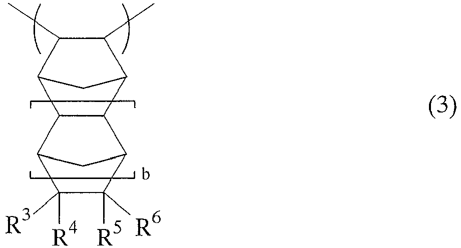 wherein R³ to R⁶ are independently selected from the group consisting of
hydrogen atoms, halogen atoms,
substituents selected from C1-10 alkyl groups, alkenyl groups, cycloalkyl groups, aryl groups, alkoxy groups, aryloxy groups and halogenated hydrocarbon groups,
polar groups selected from oxetanyl groups, alkoxycarbonyl groups, polyoxyalkylene groups and polyglyceryl groups, and
alkoxysilyl groups. Any two of R³ to R⁶ may form, in conjunction with carbon atoms to which they join, an alicyclic structure, aromatic ring structure, carbonimide group, or acid anhydride. b is an integer of 0 to 2.

The rate of the repeating unit (3) is preferably 5 to 60%, more preferably 10 to 40%, of all the repeating units. With this ratio lower than 5%, the resulting polymer may have a low molecular weight and hence gives no coating film. With this ratio higher than 60%, the resulting polymer may not produce the desired effect attributable to the group represented by X in the formula (1).

The polymer should preferably have a number-average molecular weight of 10,000 to 2,000,000, more preferably 100,000 to 800,000 (in terms of polystyrene) determined by GPC (gel permeation chromatography). With a molecular weight higher than the foregoing upper limit, the polymer may give a viscous solution which makes the resulting cosmetic product feel sticky. On the other hand, with a molecular weight lower than the foregoing lower limit, the polymer may not form coating film easily.

### (II) Making method of polymer

The polymer can be produced by addition polymerization from the compounds represented by the formulas (6) and (7). 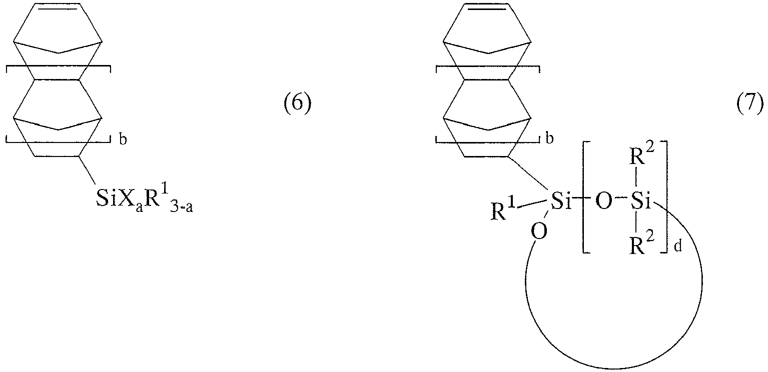 wherein R¹, R², X, a, b, and d are defined as above.

Those compounds represented by the formulas (6) and (7) are exemplified as following (in which Me is a methyl group). They may be used in combination with one another.

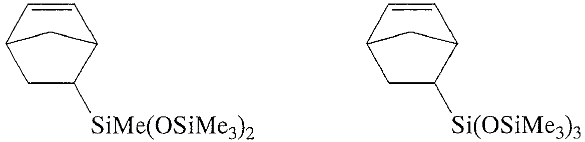

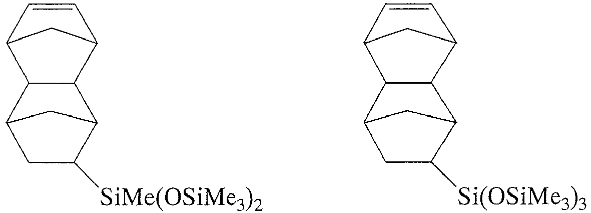

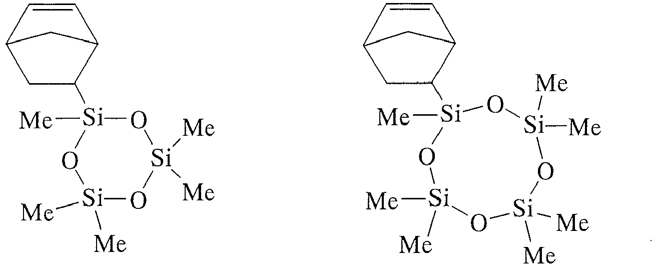

The compound represented by the formula (6) can be produced by Diels-Alder reaction from cyclopentadiene and tristrimethylsiloxyvinylsilane or the like which is a compound having a vinyl group as represented by the following (8). The compound represented by the formula (7) can be produced by Diels-Alder reaction from cyclopentadiene and a compound having a vinyl group as represented by the following formula (9). A detailed description of the production process will be given in Examples later.

(CH₂=CH) SiXₐR¹₃₋ₐ (8)

wherein R¹, X, and a are defined as above. 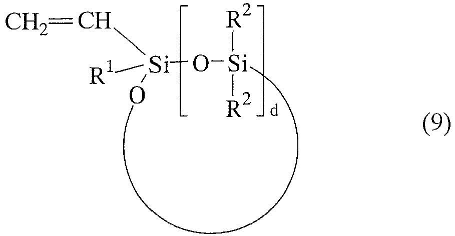 wherein R¹, R², and d are defined as above.

The polymer containing the repeating unit represented by the formula (3) can be obtained by addition polymerization from the compound represented by the formula (10) and the compound represented by the formula (6) and/or (7). The amounts of the reactants should be established so that the resulting polymer contains the repeating unit represented by the formula (3) in the ratio specified above. 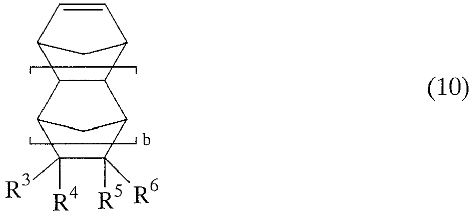 wherein R³ to R⁶, and b are defined as above.

Any two of R³ to R⁶ may form, in conjunction with carbon atoms to which they join, an alicyclic structure, aromatic ring structure, carbonimide group, or acid anhydride. Examples of the alicyclic structure may have 4 to 10 carbon atoms. They have the structure as exemplified as following, in which Me is a methyl group and Ph is a phenyl group.

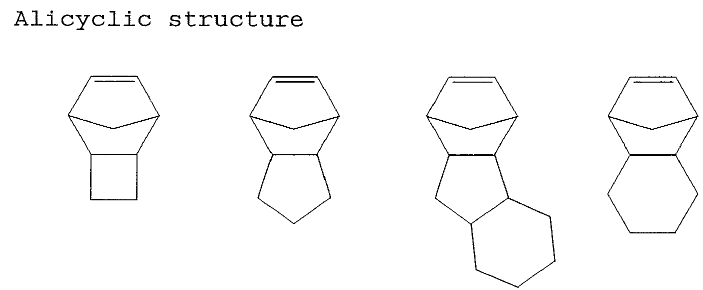

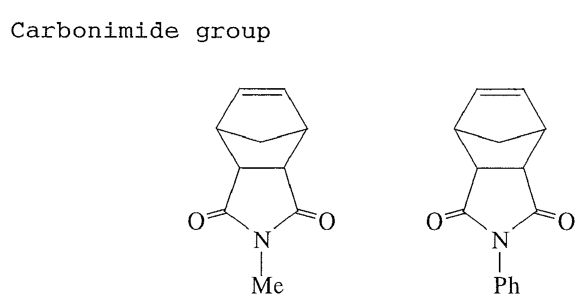

The addition polymerization for the compounds represented by the formulas (6), (7), and (10) may be accomplished in the usual way under normal pressure at 20 to 80°C in the presence of a polymerization catalyst and cocatalyst with stirring in an inert gas atmosphere, with said compounds (as monomers) dissolved in an aromatic hydrocarbon solvent such as toluene and xylene. The polymerization catalyst may be any of metal complexes containing an element selected from the 8th, 9th, and 10th groups of the periodic table. Such elements include iron (Fe), cobalt (Co), nickel (Ni), ruthenium (Ru), rhodium (Rh), palladium (Pd), and platinum (Pt). Preferred metal complexes include metallocene compounds and β-diketone compounds of nickel (Ni) or palladium (Pd). The cocatalyst includes organoaluminum compounds and ionic boron compounds. Preferred organoaluminum compounds include methylaluminoxane and preferred ionic boron compounds include triphenylcarbenium tetrakis(pentafluorophenyl) borate.

The amount of the catalyst and cocatalyst mentioned above should preferably be 0.001 to 100 mmol atom for 1 mol of the total amount of the monomers represented by the formulas (1), (2), and (3).

The polymerization system may be incorporated with a molecular weight modifier according to need. The molecular weight modifier includes, for example, hydrogen, α-olefins (such as ethylene, butene, hexene, and octene), aromatic vinyl compounds such as styrene, 3-methylstyrene, and divinyl benzene, unsaturated ethers such as ethyl vinyl ether, and vinyl silicon compounds such as tris(trimethylmethoxy)vinylsilane, divinyldihydrosilane, and vinylcyclotetrasiloxane.

The ratio of solvent to monomer, the polymerization temperature, the polymerization time, and the amount of the molecular weight modifier widely vary depending on the catalyst employed and the structure of the monomers. Hence, they cannot be established unconditionally. They should be properly specified according to the structure of the intended polymer. The molecular weight of the polymer may be adjusted by the amount of the polymerization catalyst, the amount of the molecular weight modifier, the ratio of conversion from monomer to polymer, and the polymerization temperature.

The polymerization is suspended by adding any compound selected from water, alcohol, ketone, and organic acid. The polymerization solution may be given a mixture of alcohol and aqueous solution of acid such as lactic acid, malic acid, and oxalic acid to separate and remove catalyst residues from it. It is also possible to remove catalyst residues by adsorption with activated carbon, diatomaceous earth, alumina, or silica, or by filtration.

The resulting polymer coagulates as the polymerization solution is put in an alcohol such as methanol and ethanol or ketone such as acetone and methyl ethyl ketone. The resulting coagulation is separated by drying under reduced pressure at 60 to 150° C for 6 to 48 hours. This step also removes catalyst residues and unreacted monomers from the polymerization solution. Unreacted monomers containing the above-mentioned siloxane can be easily removed by using a solvent composed of alcohol or ketone and cyclic polysiloxane such as octamethylcyclotetrasiloxane and decamethylcyclopentasiloxane.

### (III) Cosmetic product

The above-mentioned polymer (which will be referred to as ingredient-A hereinafter) is useful as a raw material for cosmetic products to be externally applied to the skin, hair, eyebrows, and eyelash. Such cosmetic products include skin care cosmetics, make-up cosmetics, hair care cosmetics, antiperspirants, and UV care cosmetics. It is useful particularly for skin care cosmetics and make-up cosmetics which tend to cause problems with long lasting performance and secondary sticking. Ingredient-A is used in an amount of 0.1 to 10 weight% in the total amount of powder cosmetic products such as foundation or in an amount of 0.1 to 7.5 weight% in the total amount of liquid cosmetic products such as milky lotion. The foregoing amount varies depending on the kind and formulation of individual cosmetic products.

The cosmetic product may be incorporated with one or more than one kind of oil as ingredient-B. This oil may be in the form of solid, semi-solid, or liquid so long as it is usable for ordinary cosmetic products.

The oil as ingredient-B includes, for example, silicone oils, hydrocarbon oils, higher fatty acids, higher alcohol oils, ester oils, glyceride oils, animal and vegetable oils, semi-synthetic oils, and fluorine-derived oils. They may be used alone or in combination with one another.

Examples of the silicone oils include, for example, straight-chain or branched-chain organopolysiloxanes ranging from low to high in viscosity such as dimethylpolysiloxane, caprylyl methicone, phenyl trimethicone, methylphenylpolysiloxane, methylhexylpolysiloxane, methylhydrogen polysiloxane, and dimethylsiloxane-methylphenylsiloxane copolymer, cyclic organopolysiloxanes such as octamethylcyclotetrasiloxane, decamethylcyclopentasiloxane, dodecamethylcyclohexasiloxane, tetramethyltetrahydrogencyclotetrasiloxane, and tetramethyltetraphenylcyclotetrasiloxane, branched-chain organopolysiloxane such as tristrimethylsiloxymethylsilane and tetrakistrimethylsiloxysilane, amino-modified organopolysiloxane, pyrrolidone-modified organopolysiloxane, pyrrolidone carboxylic acid-modified organopolysiloxane, silicone rubber such as gum-like dimethylpolysiloxane having a high degree of polymerization, gum-like amino-modified organopolysiloxane, and gum-like dimethylsiloxane-methylphenylsiloxane copolymer, solution of silicone rubber or gum in cyclic organopolysiloxane, trimethylsiloxysilicic acid, titanium oxide-containing trimethylsiloxysilicic acid, solution of trimethylsiloxysilicic acid in cyclic organopolysiloxane, higher alkoxy-modified organopolysiloxane such as stearoxysilicone, higher fatty acid-modified organopolysiloxane, alkyl-modified organopolysiloxane, long-chain alkyl-modified organopolysiloxane, fluorine-modified organopolysiloxane, silicone resin, and solution of silicone resin.

A portion of the oil as ingredient-B should preferably be silicone oil (volatile at room temperature 25°C) or isododecane. Examples of the former include linear, branched, and cyclic ones, such as octamethyltrisiloxane, decamethyltetrasiloxane, octamethylcyclotetrasiloxane, decamethylcyclopentasiloxane, tristrimethylsiloxymethylsilane, tetrakistrimethylsiloxysilane, and dodecamethylpentasiloxane. It is desirable that ingredient-A should be highly compatible with these silicone oils and should be used in the form of their solution prepared before addition to the cosmetic product.

The hydrocarbon oils include straight-chain ones, branched-chain ones, and volatile ones. They are exemplified by ozokerite, α-olefin oligomers, hexane, heptane, octane, cyclohexane, toluene, benzene, xylene, cyclohexene, light isoparaffin, isododecane, light liquid isoparaffin, squalane, synthetic squalane, vegetable squalane, squalene, ceresin, paraffin, paraffin wax, polyethylene wax, polyethylene-polypropylene wax, (ethylene/propylene/styrene) copolymer, (butylene/propylene/styrene) copolymer, liquid paraffin, liquid isoparaffin, pristane, polyisobutylene, hydrogenated polyisobutene, microcrystalline wax, and vaseline.

Examples of the higher fatty acid include lauric acid, myristic acid, palmitic acid, stearic acid, behenic acid, undecylenic acid, oleic acid, linoleic acid, linolenic acid, arachidonic acid, eicosapentaenoic acid (EPA), docosahexaenoic acid (DHA), isostearic acid, and 12-hydroxystearic acid.

Examples of the higher alcohol oil include lauryl alcohol, myristyl alcohol, palmityl alcohol, stearyl alcohol, behenyl alcohol, hexadecyl alcohol, oleyl alcohol, isostearyl alcohol, hexyldodecanol, octyldodecanol, cetostearyl alcohol, 2-decyltetradecinol, cholesterol, phytosterol, POE cholesterol ether, monostearyl glycerine ether (batyl alcohol), and monooleyl glyceryl ether (selachyl alcohol).

Examples of the ester oils include diisobutyl adipate, 2-hexyldecyl adipate, di-2-heptylundecyl adipate, N-alkylglycol monoisostearate, isocetyl isostearate, trimethylolpropane triisostearate, ethyleneglycol di-2-ethylhexanoate, cetyl 2-ethylhexanoate, trimethylolpropane tri-2-ethylhexanoate, pentaerythritol tetra-2-ethylhexanoate, cetyl octanoate, octyldodecyl gum ester, oleyl oleate, octyldodecyl oleate, decyl oleate, neopentylglycol dioctanoate, neopentylglycol dicaprylate, triethyl citrate, 2-ethylhexyl succinate, amyl acetate, ethyl acetate, butyl acetate, isocetyl stearate, butyl stearate, diisopropyl sebacate, di-2-ethylhexyl sebacate, cetyl lactate, myristyl lactate, isononyl isononanoate, isotridecyl isononanoate, isopropyl palmitate, 2-ethylhexyl palmitate, 2-hexyldecyl palmitate, 2-heptylundecyl palmitate, cholesteryl 12-hydroxystearylate, dipentaerythritol fatty acid ester, isopropyl myristate, octyldodecyl myristate, 2-hexyldecyl myristate, myristyl myristate, hexyldecyl dimethyloctanoate, ethyl laurate, hexyl laurate, N-lauroyl-L-glutamic acid-2-octyldodecyl ester, lauroylsarcosine isopropyl ester, and diisostearyl malate.

Examples of the glyceride oils include acetoglyceryl, glyceryl triisooctanoate, glyceryl triisostearate, glyceryl triisopalmitate, glyceryl monostearate, glyceryl di-2-heptylundecanoate, glyceryl trimyristate, and diglyceryl myristate and isostearate.

Examples of the animal and vegetable oils and semisynthetic oils include avocado oil, linseed oil, almond oil, purified insect wax, perilla oil, olive oil, cacao butter, kapok wax, kaya nut oil, carnauba wax, liver oil, candelilla wax, purified candelilla wax, beef tallow, neat's foot oil, neat's bone oil, hardened beef tallow, apricot kernel oil, whale oil, hardened oil, wheat germ oil, sesame oil, rice germ oil, rice barn oil, sugarcane wax, sasanqua oil, sunflower oil, shea butter, china wool oil, cinnamon oil, jojoba wax, squalane, squalene, shellac wax, turtle oil, soybean oil, tea seed oil, camellia oil, evening primrose oil, corn oil, lard, rapeseed oil, Japanese tung oil, rice bran wax, germ oil, horse tallow, persic oil, palm oil, palm kernel oil, castor oil, hardened castor oil, castor oil fatty acid methyl ester, sunflower oil, grape seed oil, bayberry wax, jojoba oil, macademianut oil, bees wax, mink oil, mink wax, meadowfoam oil, cottonseed oil, cotton wax, Japanese wax, Japanese wax kernel oil, montan wax, coconut oil, hardened coconut oil, coconut fatty acid triglyceride, mutton tallow, peanut oil, lanolin, liquid lanolin, reduced lanolin, lanolin alcohol, lanolin wax, hard lanolin, lanolin acetate, lanolin acetate alcohol, lanolin fatty acid isopropyl, POE lanolin alcohol ether, POE lanolin alcohol acetate, lanolin fatty acid polyethylene glycol, POE hydrogenated lanolin alcohol ether, and egg yolk oil. (POE stands for polyoxyethylene.)

Examples of the fluorine-containing oils include perfluoropolyoxyalkylene, perfluorodecalin, and perfluorooctane.

The amount of ingredient-B should be 1 to 98 weight% in the total amount of the cosmetic product. It should be properly adjusted according to the formulation of the cosmetic product. The above-mentioned silicone oil which is volatile at 25°C and of straight-chain, branched-chain, or cyclic structure should be added in such an amount that the ratio (by mass) of the silicone oil to the polymer as ingredient-A is from 1:2 to 100:1, preferably from 1:1 to 70:1.

The cosmetic product according to the present invention may be incorporated with one or more than one kind of compound (as ingredient-C) which contains an alcoholic hydroxyl group in its molecular structure. Examples of such a compound include lower alcohol such as ethanol, isopropanol, and butanol, sugar alcohol such as sorbitol and maltose, sterol such as cholesterol, sitosterol, phytosterol, and lanosterol, and polyhydric alcohol such as butylene glycol, propylene glycol, dibutylene glycol, and pentylene glycol. Common examples of ingredient-C are water-soluble monohydric alcohols and water-soluble polyhydric alcohols. The amount of ingredient-C should be 0.1 to 98 weight% in the total amount of the cosmetic product.

The cosmetic product according to the present invention may be incorporated with one or more than one kind of water-soluble polymeric compound or water-swelling polymeric compound as ingredient-D that suits its intended use. Examples of the water-soluble polymeric compound are listed as following. Polymeric compounds of vegetable origin, such as acacia, tragacanth, galactan, carob gum, guar gum, karaya gum, carrageenan, pectin, agar-agar, quince seed, starch (rice, corn, potato, wheat, etc.), algecolloid, trant gum, and locust bean gum. Polymeric compounds of microbe origin, such as xanthan gum, dextran, succinoglucan, and pullulan. Polymeric compounds of animal origin, such as collagen, casein, albumin, and gelatin. Polymeric compounds derived from starch, such as carboxymethyl starch and methylhydroxypropyl starch. Polymeric compounds derived from cellulose, such as methyl cellulose, ethyl cellulose, methylhydroxypropyl cellulose, carboxymethyl cellulose, hydroxymethyl cellulose, hydroxypropyl cellulose, nitrocellulose, cellulose sodium sulfate, carboxymethyl cellulose sodium, crystalline cellulose, cellulose acetate, and cellulose powder. Polymeric compounds derived from alginic acid, such as sodium alginate and propylene glycol alginage ester. Polymeric compounds having vinyl groups, such as polyvinyl methyl ether and carboxyvinyl polymer. Polymeric compounds of polyoxyethylene. Polymeric compounds of polyoxyethylene-polyoxypropylene copolymer. Acrylic polymeric compounds, such as sodium polyacrylate, polyethyl acrylate, polyacrylamide, and acryloyldimethyl taulin salt copolymer. Other synthetic water-soluble polymeric compounds, such as polyethylene imine and cationic polymer. Inorganic polymeric compounds, such as bentonite, aluminum-magnesium silicate, montmorillonite, beidellite, nontronite, saponite, hectorite, and silicic anhydride. In addition, the foregoing water-soluble polymeric compounds include such film-forming agents as polyvinyl alcohol and polyvinyl pyrrolidone. They should be added in an amount of 0 to 25 weight% in the total amount of the cosmetic product.

The cosmetic product according to the present invention may be incorporated with water as ingredient-E. The amount of water, which varies depending on formulation, should be 1 to 99 weight% in the total amount of the cosmetic product.

The cosmetic product according to the present invention may be incorporated with a powder as ingredient-F. So long as being suitable for ordinary cosmetic products, this powder is not specifically restricted in shape (spherical, acicular, placoid, dendroid, fibrous, or amorphous), particle size (fume, microsphere, or pigment), and particle structure (porous, nonporous, hollow, or hollow porous). Examples of such powder include inorganic powder, organic powder, powder of metal salt of surface active agent, colored pigment, pearlescent pigment, metal powder pigment, tar dye, and natural color.

Examples of the inorganic powder include titanium oxide, zirconium oxide, zinc oxide, cerium oxide, magnesium oxide, barium sulfate, calcium sulfate, magnesium sulfate, calcium carbonate, magnesium carbonate, talk, mica, kaolin, cerisite, white mica, synthetic mica, bronze mica, lepidolite, black mica, lithia mica, silicic acid, silicic acid anhydride, aluminum silicate, magnesium silicate, aluminum magnesium silicate, calcium silicate, barium silicate, strontium silicate, metal tungstate, hydroxyapatite, vermiculite, higilite, bentonite, montmorillonite, hectolite, zeolite, ceramics powder, dibasic calcium phosphate, alumina, aluminum hydroxide, boron nitride, silicon nitride, silica, and silylated silica. Of these examples, zinc oxide and titanium oxide are preferable. In addition, calcium carbonate and talc, which are extender pigments, may also be used preferentially. The powders listed above are made to easily disperse into the cosmetic product containing ingredient-A.

Examples of the organic powders include polyamide powder, polyacrylic acid-acrylate ester powder, polyester powder, polyethylene powder, polypropylene powder, polystyrene powder, polyurethane powder, benzoguanamine powder, polymethylbenzoguanamine powder, tetrafluoroethylene powder, polymethyl methacrylate powder, cellulose powder, silk powder, powder or fibrous powder of nylon such as 12-nylon and 6-nylon, crosslinked silicone fine powder prepared by crosslinking dimethylpolysiloxane, spherical fine powder of crosslinked polymethylsilsesquioxane, spherical fine powder of crosslinked organopolysiloxane rubber (having its surface coated with polymethylsilsesquioxane), hydrophobic silica, fine laminated powder or crystalline fibrous powder of resins such as styrene-acrylic acid copolymer, divinylbenzene-styrene copolymer, vinyl resin, urea resin, phenolic resin, fluoroplastic resin, silicone resin, acrylic resin, melamine resin, epoxy resin, and polycarbonate resin, starch powder, fatty acid starch derivative powder, and lauroyl lysine.

Preferable among these examples are fine powder of crosslinked spherical dimethylpolysiloxane (which partly has the crosslinked structure of dimethylpolysiloxane), fine powder of crosslinked spherical polymethylsilsesquioxane, fine powder of crosslinked spherical dimethylpolysiloxane rubber (having its surface coated with polymethylsilsesquioxane), fine powder of crosslinked spherical diphenylpolysiloxane rubber (having its surface coated with polymethylsilsesquioxane), and hydrophobic silica. They are commercially available from Shin-Etsu Chemical Co., Ltd. under the trade names of KMP-590, KSP-100, KSP-101, KSP-102, KSP-105, and KSP-300.

Examples of the powder of metal salt of surface active agent include metal soaps, such as zinc stearate, aluminum stearate, calcium stearate, magnesium stearate, zinc myristate, magnesium myristate, zinc cetylphosphate, calcium cetylphosphate, zinc sodium cetylphosphate, zinc palmitate, aluminum palmitate, and zinc laurate.

The coloring agents include pigments and dyes. Examples of the colored pigments include inorganic red pigments such as iron oxide, iron hydroxide, and iron titanate, inorganic brown pigments such as γ-iron oxide, inorganic yellow pigments such as yellow iron oxide and Chinese yellow, inorganic black pigments such as black iron oxide and carbon black, inorganic violet pigments such as silica-coated iron pigment, manganese violet, and cobalt violet, inorganic green pigment such as chromium hydroxide, chromium oxide, cobalt oxide, and cobalt titanate, inorganic blue pigment such as prussian blue and ultramarine blue, laked tar dye, laked natural dye, and synthetic resin powder formed by combination of the foregoing powders.

Examples of the pearlescent pigments include mica coated with titanium oxide, bismuth oxychloride, bismuth oxychloride coated with titanium oxide, talk coated with titanium oxide, silica-coated mica titanium, fish scale guanine, and colored mica with titanium oxide coating.

Examples of the metal powders include aluminum powder, copper powder, and stainless steel powder.

Examples of the tar dyes include red 3, red 104, red 106, red 201, red 202, red 204, red 205, red 220, red 226, red 227, red 228, red 230, red 401, red 505, yellow 4, yellow 5, yellow 202, yellow 203, yellow 204, yellow 401, blue 1, blue 2, blue 201, blue 404, green 3, green 201, green 204, green 205, orange 201, orange 203, orange 204, orange 206, and orange 207. Examples of the natural dyes include carminic acid, laccaic acid, carthamin, brazilin, and crocin.

Additional examples of the powders include fine powder of titanium oxide, fine powder of iron-containing titanium oxide, fine powder of zinc oxide, fine powder of cerium oxide, and a combination thereof, which absorb or scatter ultraviolet rays. These powders may have surface treatment with aluminum oxide, aluminum hydroxide, silica, silica gel, zinc silicate, or the like, for the purpose of blocking the surface activity of the powder.

The foregoing powders may be combined with other powders or surface-treated with an adequate material to such an extent that the present invention is not adversely affected. Examples of the surface-treatment materials include ordinary oils, silicone oils, fluorine compounds, surface active agents, and metal soaps. Additional examples are listed as following. Alkyl group having a hydrogen atom directly attaching to a silicon atom or a hydrolyzable silyl group. Linear and/or branched organopolysiloxane having a hydrogen atom directly attaching to a silicon atom or a hydrolyzable silyl group. Linear and/or branched organopolysiloxane (modified with long-chain alkyl groups) having a hydrogen atom directly attaching to a silicon atom or a hydrolyzable silyl group. Linear and/or branched organopolysiloxane (modified with polyoxyalkylene) having a hydrogen atom directly attaching to a silicon atom or a hydrolyzable silyl group. Acrylsilicone copolymer having a hydrogen atom directly attaching to a silicon atom or a hydrolyzable silyl group. They may be used alone or in combination with one another according to need. These powders may be used after surface treatment with any of AES-3083, KF-99P, KF-9901, KF-9908, KF-9909, and KP-574, all available from Shin-Etsu Chemical Co., Ltd.

The cosmetic product according to the present invention may be incorporated with a surface active agent as ingredient-G. The surface active agent falls under the category of anionic, cationic, nonionic, and amphoteric. They are not specifically restricted so long as they are intended for cosmetic use.

Examples of the anionic surface active agents include fatty acid soaps such as sodium stearate, potassium stearate, triethanolamine stearate, sodium palmitate, potassium palmitate, triethanolamine palmitate, sodium laurate, potassium laurate, and triethanolamine laurate; alkylether carboxylic acid and salt thereof; salt of condensate of amino acid and fatty acid; alkane sulfonate, alkene sulfonate, sulfonate of fatty acid ester, sulfonate of fatty acid amide, sulfonate of formalin condensate, alkyl sulfate ester salt, secondary higher alcohol sulfate ester salt, alkyl and allyl ether sulfate ester salt, sulfate ester salt of fatty acid ester, sulfate ester salt of fatty acid alkylolamide, sulfate ester salt such as sulfated castor oil, alkyl phosphate, ether phosphate, alkylallyl ether phosphate, amide phosphate, N-acyl lactate, N-acylsarcosine salt, and N-acylamino acid surfactant. Examples of the cationic surface active agents include amine salts such as alkylamine salt, and polyamine and aminoalcohol fatty acid derivatives, alkyl quaternary ammonium salt, aromatic quaternary ammonium salt, pyridinium salt, and imidazolium salt.

Examples of the nonionic surface active agents include sorbitan fatty acid ester, glycerin fatty acid ester, polyglycerin fatty acid ester, propylene glycol fatty acid ester, polyethylene glycol fatty acid ester, sucrose fatty acid ester, methylglucoside fatty acid ester, alkylpolyglucoside, polyoxyethylene alkyl ether, polyoxypropylene alkyl ether, polyoxyethylene alkyl phenyl ether, polyoxyethylene fatty acid ester, polyoxyethylene sorbitan fatty acid ester, polyoxyethylene sorbitol fatty acid ester, polyoxyethylene glycerin fatty acid ester, polyoxyethylene propylene glycol fatty acid ester, polyoxyethylene castor oil, polyoxyethylene hardened castor oil, polyoxyethylene phytostanol ether, polyoxyethylene phytosterol ether, polyoxyethylene cholestanol ether, polyoxyethylene cholesteryl ester, linear or branched polyoxyalkylene-modified organopolysiloxane, linear or branched polyoxyalkylene-alkyl comodified organopolysiloxane, linear or branched polyglycerin-modified organopolysiloxane, linear or branched polyglycerin-alkyl comodified organopolysiloxane, alkanolamide, sugar ether, and sugar amide. Examples of the amphoteric surface active agents include betaine, phosphatidylcholine, salt of aminocarboxylic acid, imidazoline derivatives, and those of amideamine type.

Preferable among the foregoing surface active agents are linear or branched organopolysiloxane having polyoxyethylene chains in the molecule, linear or branched organopolysiloxane having polyglycerin chains in the molecule, and their alkyl-modified organopolysiloxane. They are commercially available from Shin-Etsu Chemical Co., Ltd. under the trade names of KF-6011, KF-6011P, KF-6043, KF-6012, KF-6013, KF-6015, KF-6016, KF-6017, KF-6028, KF-6028P, KF-6038, KF-6100, KF-6104, and KF-6105. They should preferably have an HLB value of 1 to 10. The amount of ingredient-G should be 0.1 to 20 weight%, preferably 0.2 to 10 weight%, in the total amount of the cosmetic product.

The cosmetic product according to the present invention may be incorporated with one or more than one kind of crosslinked organopolysiloxane as ingredient-H if necessary for its intended use. The crosslinked organopolysiloxane should preferably be one which swells upon absorption of liquid oil more than its own weight. The liquid oil may be the one mentioned above as ingredient-B.
It includes, for example, low-viscosity silicone oils having a viscosity of 0.65 to 100.0 mm²/s (at 25°C), liquid paraffin, squalane, hydrocarbon oil such as isododecane, glyceride oil such as trioctanoin, ester oil such as isotridecyl isononanoate, N-acrylglutamate ester, and lauroyl sarcosinate ester, and natural animal and vegetable oils such as macadamia nut oil.

It is desirable that the crosslinked organopolysiloxane be crosslinked by a crosslinking agent which has more than one vinyl reaction site in the molecule and forms the crosslinked structure upon reaction between hydrogen atoms each joining directly to a silicon atom. Examples of the crosslinking agent include organopolysiloxane having more than one vinyl group in the molecule, polyoxyalkylene having more than one allyl group in the molecule, polyglycerin having more than one allyl group in the molecule, and α,ω-alkenyldiene.

In addition, the crosslinked organopolysiloxane may contain a moiety selected from the group composed of polyoxyalkylene moiety, polyglycerin moiety, alkyl moiety, alkenyl moiety, aryl moiety, and fluoroalkyl moiety. The crosslinked organopolysiloxane is not specifically restricted, and it is commercially available from Shin-Etsu Chemical Co., Ltd. under the trade names of KSG-15, KSG-16, KSG-18, KSG-1610, USG-103, KSG-210, KSG-240, KSG-710 (in the form of paste with silicone oil), USG-106, KSG-41, KSG-42, KSG-43, KSG-44, KSG-310, KSG-320, KSG-330, KSG-340, KSG-810, KSG-820, KSG-830, KSG-840 (in the form of paste with hydrocarbon oil or triglyceride oil).

The amount of ingredient-H should be 0.01 to 40 weight%, preferably 0.1 to 30 weight%, in the total amount of the cosmetic product.

The cosmetic product according to the present invention may be incorporated with one or more than one kind of silicone resin, which is either acrylsilicone resin or silicone resin with network structure. The acrylsilicone resin is an acryl/silicone graft or block copolymer. It also includes acrylsilicone resins which contain in the molecule a moiety selected from the group composed of pyrrolidone moiety, long-chain alkyl moiety, polyoxyalkylene moiety, fluoroalkyl moiety, and anionic moiety such as carboxylic acid. It is commercially available from Shin-Etsu Chemical Co., Ltd. under the trade names of KP-541, KP-543, KP-545, KP-549, KP-550, KP-571, KP-575, and KP-581 (dissolved in silicone oil or hydrocarbon oil).

The silicone resin with network structure is a network compound of silicone which is represented by MQ, MDQ, MT, MTQ, MDT, or MDTQ, where M, D, T, and Q are respectively R₃SiO_{0.5} unit, R₂SiO unit, RSiO_{1.5} unit, and SiO₂ unit. It is typically exemplified by trimethylsiloxysilicic acid. The silicone network resin is known as MQ resin, MT resin, or MDT resin.
It may also have moieties indicated by MDQ, MTQ, or MDTQ. It is commercially available in the form of solution in decamethylcyclopentasiloxane or isododecane. It may contain in the molecule a moiety selected from the group composed of pyrrolidone moiety, long-chain alkyl moiety, polyoxyalkylene moiety, fluoroalkyl moiety, and amino moiety.

The silicone resin, such as acrylsilicone resin and silicone network compound, should be used in an amount of 0.1 to 20 weight%, preferably 1 to 10 weight%, in the total amount of the cosmetic product. The silicone network compound is hard and brittle and hence, when incorporated into a cosmetic product, it is subject to secondary sticking and easily peels off by rubbing. However, this disadvantage is overcome when it is added to the cosmetic product of the present invention.

The cosmetic product according to the present invention may be incorporated with silicone wax as ingredient-J for its intended use. This silicone wax should preferably be a polylactone-modified polysiloxane in which the polylactone is a ring-opening polymerizate of lactone compound of ring structure with five or more members. It should preferably be an acryl-modified polysiloxane containing in the molecule a moiety selected from the group composed of pyrrolidone moiety, long-chain alkyl moiety, polyoxyalkylene moiety, fluoroalkyl moiety, and anion moiety such as carboxylic acid. It is commercially available from Shin-Etsu Chemical Co., Ltd. under the trade names of KP-561P and KP-562P as wax containing long-chain alkyl group.

Moreover, the above-mentioned silicone wax should preferably be silicone-modified olefin wax which is obtained by addition reaction from olefin wax and organohydrogenpolysiloxane having more than one SiH linkage in one molecule. The olefin wax should preferably be one which is obtained by copolymerization from ethylene and at least one species of diene or one which is obtained by copolymerization from ethylene, at least one species of olefin selected from C3-12 α-olefins, and at least one species of diene. A preferred diene is vinylnorbornene.

The silicone wax, no matter what, should be used in an amount of 0.1 to 20 weight%, preferably 1 to 10 weight%, in the total amount of the cosmetic.

The cosmetic product according to the present invention may be incorporated with any ingredient listed as following, which is used for ordinary cosmetic products, in an amount not harmful to the effect of the present invention. Oil-soluble gelling agent, organo-modified clay mineral, resin, antiperspirant, UV absorber, UV absorbing-scattering agent, moisturizer, antibacterial agent, perfume, salt, antioxidant, pH adjuster, chelating agent, refrigerant, antiinflammatory agent, skin care agent (whitening agent, cell activating agent, rough skin improving agent, blood flow stimulant, skin astringent, antiseborrheic agent, etc.), vitamin, amino acid, nucleic acid, hormone, inclusion compound, and hair fixing agent.

Examples of the oil-soluble gelling agent include the following. Metal soaps such as aluminum stearate, magnesium stearate, and zinc myristate; amino acid derivatives such as N-lauroyl-L-glutamic acid-α,γ-di-n-butylamine; dextrin fatty acid ester such as dextrin palmitic acid ester, dextrin stearic acid ester, and dextrin 2-ethylhexanoic acid palmitic acid ester; sucrose fatty acid ester such as sucrose palmitic acid ester and sucrose stearic acid ester; fructo-oligosaccharide fatty acid ester such as fructo-oligosaccharide stearic acid ester and fructo-oligosaccharide 2-ethylhexanoic acid ester; benzylidene derivative of sorbitol such as monobenzylidene sorbitol and dibenzylidene sorbitol; and organo-modified clay mineral such as dimethylbenzyldodecyl ammonium montmorillonite clay and dimethyldioctadecyl ammonium montmorillonite clay.

Examples of the antiperspirant include aluminum chlorohydrate, aluminum chloride, aluminum sesquichlorohydrate, zirconyl hydroxychloride, aluminum zirconium hydroxychloride, and aluminum zirconium glycine complex.

Examples of the UV absorber include those based on benzoic acid such as para-aminobenzoic acid, those based on anthranilic acid such as methyl anthranilate, those based on salicylic acid such as methyl salicylate, octyl salicylate, and trimethylcyclohexyl salicylate, those based on cinnamic acid such as octyl paramethoxycinnamate, those based on benzophenone such as 2,4-dihydroxybenzophenone, those based on urocanic acid such as ethyl urocanate, those based on dibenzoylmethane such as 4-t-butyl-4'-methoxybenzoylmethane, and those based on phenylbenzimidazole sulfonic acid. Examples of the UV absorbing-scattering agent include fine powder of titanium oxide, fine powder of iron-containing titanium oxide, fine powder of zinc oxide, fine powder of cerium oxide, and their combination, which absorb and scatter UV rays. These fine powders may be used in the form of dispersion in oil.

Examples of the moisturizers include glycerin, sorbitol, propylene glycol, dipropylene glycol, 1,3-butylene glycol, pentylene glycol, glucose, xylitol, maltitol, polyethylene glycol, hyaluronic acid, chondroitin sulfate, pyrrolidone carboxylate, polyoxyethylene methyl glucoside, polyoxypropylene methyl glucoside, egg yolk lecithin, soybean lecithin, phosphatidylcholine, phosphatidylethanolamine, phosphatidylserine, phosphatidylglycerol, phosphatidylinositol, and sphingophospholipid.

Examples of the antibacterial agents include paraoxybenzoate alkyl ester, benzoic acid, sodium benzoate, sorbic acid, potassium sorbate, phenoxyethanol, salicylic acid, phenol, parachorometacresol, hexachlorophene, benzalkonium chloride, chlorohexidine chloride, trichlorocarbanilide, and photosensitive substance.

Examples of the salts include inorganic salts, organic salts, amine salts, and amino acid salts. Examples of the inorganic salts include sodium, potassium, magnesium, calcium, aluminum, zirconium, and zinc salts of inorganic acids such as hydrochloric acid, sulfuric acid, carbonic acid, and nitric acid. Examples of the organic salts include salts of organic acids such as acetic acid, dehydroacetic acid, citric acid, malic acid, succinic acid, ascorbic acid, and stearic acid. Examples of the amine salts and amino acid salts include salts of amines such as triethanolamine and salts of amino acids such as glutamic acid. Additional examples include salts of hyaluronic acid and chondroitin sulfuric acid, aluminum zirconium glycine complex, and acid-alkali neutralized salts which are commonly used for cosmetic formulations.

Examples of the antioxidants include tocopherol, p-t-butylphenol, butylhydroxyanisole, dibutylhydroxytoluene, and phytic acid. Examples of the pH adjuster include lactic acid, citric acid, glycolic acid, succinic acid, tartaric acid, dl-malic acid, potassium carbonate, sodium hydrogen carbonate, and ammonium hydrogen carbonate. Examples of the chelating agents include alanine, sodium edetate, sodium polyphosphate, sodium metaphosphate, and phosphoric acid. Examples of the refrigerants include L-menthol and camphor. Examples of the antiinflammatory agents include allantoin, glycyrrhizic acid and salt thereof, glycyrrhetic acid, stearyl glycyrrhetinate, tranexamic acid, and azulene.

Examples of the skin care agents include whitening agents such as placenta extract, albutin, glutathione, and saxifraga sarmentosa extract, cell activating agent such as royal jelly, photosensitive substance, cholesterol derivatives, and calf blood extract, skin roughness-improving agent, blood flow stimulant such as nonylic vanillamide, nicotinic benzyl ester, nicotinic β-butoxyethyl ester, capsaicin, zingerone, cantharis tincture, ichthammol, caffeine, tannic acid, α-borneol, tocopherol nicotinate, inositol hexanicotinate, cyclandelate, cinnarizine, tolazoline, acetylcholine, verapamil, cepharanthine, and γ-oryzanol, skin astringent such as zinc oxide and tannic acid, and antiseborrheic agent such as sulfur and thiantrol.

Examples of the vitamins include vitamin A analogs such as vitamin A oil, retinol, retinol acetate, and retinol palmitate, vitamin B₂ analogs such as riboflavin, riboflavin butyrate, and flavinadenine nucleotide, vitamin B₆ analogs such as pyridoxine hydrochloride, pyridoxine dioctanoate, and pyridoxine tripalmitate, vitamin B analogs such as vitamin B₁₂ and derivatives thereof, and vitamin B₁₅ and derivatives thereof, vitamin C analogs such as L-ascorbic acid, ester of L-ascorbic acid dipalmitic acid, sodium salt of L-ascorbic acid-2-sulfuric acid, and diester and dipotassium salt of L-ascorbic acid phosphoric acid, vitamin D analogs such as ergocalciferol and cholecalciferol, vitamin E analogs such as α-tocopherol, β-tocopherol, γ-tocopherol, dl-α-tocopherol acetate, dl-α-tocopherol nicotinate, and dl-α-tocopherol succinate, vitamin H, vitamin P, nicotinic acid analogs such as nicotinic acid, benzyl nicotinate, and nicotinic amide, pantothenoic acid analogs such as calcium pantothenate, D-pantothenyl alcohol, pantothenyl ethyl ether, and acetylpantothenyl ethyl ether, and biotin.

Examples of the amino acids include glycine, valine, leucine, isoleucine, serine, threonine, phenylalanine, arginine, lysine, aspartic acid, glutamic acid, cystine, cysteine, methionine, and tryptophane. Examples of the nucleic acids include deoxyribonucleic acid. Examples of the hormones include estradiol and ethenylestradiol.

The polymeric compound to fix the cosmetic product to nails and hair includes, for example, amphoteric, anionic, cationic, and nonionic ones as listed as following. Polyvinylpyrrolidone polymers such as polyvinylpyrrolidone and vinylpyrrolidone/vinyl acetate copolymer, acidic vinyl ether polymers such as methyl vinyl ether/maleic anhydride alkyl half ester copolymer, acidic polyvinyl acetate polymers such as vinyl acetate/crotonic acid copolymer, acidic acrylic polymers such as (meth)acrylic acid/alkyl (meth)acrylate copolymer, (meth)acrylic acid/alkyl (meth)acrylate/alkylacrylamide copolymer, and amphoteric acrylic polymers such as N-methacryloylethyl-N,N-dimethylammonium·α-N-methylcarboxybetaine/alkyl (meth)acrylate copolymer and hydroxypropyl (meth)acrylate/butylaminoethyl methacrylate/acrylic acid octylamide copolymer. Additional examples include natural polymers such as cellulose and derivatives thereof, keratin, collagen, and derivatives thereof and synthetic resins such as alkyd resin, epoxy resin, phenolic resin, unsaturated polyester, acrylic copolymer, urethane resin, various rubbers such as NBR, SBR, SIS, SBS, and EPDM, and chlorinated polyethylene.

The cosmetic product according to the present invention, which is composed of the foregoing ingredients, comes in the form of powder, oil, oil-in-water emulsion, water-in-oil emulsion, non aqueous emulsion or multiemulsion (W/O/W and O/W/O). They are in the form of toilet water, milky lotion, cream, cleansing, pack, massage oil, facial lotion, facial oil, cleansing agent, deodorant, hand cream, lip cream, skin care product (to hide wrinkles), make-up base, concealer, white make-up powder, powder foundation, liquid foundation, cream foundation, oil foundation, blush-on product, eye shadow, mascara, eye liner, eye brow, lipstick, manicure products such as nail enamel, nail undercoat, and nail overcoat, hair cosmetics such as shampoo, rinse, treatment, and setting lotion, antiperspirant, and ultraviolet protective cosmetic preparations such as sunscreen oil, sunscreen milky emulsion, and sunscreen cream.

The foregoing cosmetic products may be produced in the form of liquid, milky emulsion, cream, solid, paste, gel, powder, press, layer, mousse, aerosol, spray, stick, and others.

Moreover, the foregoing cosmetic product may be applied to the skin, hair, eye brow, eye lash, and nail by means of fingers or applicators such as brush, chip, puff, and spatula.

### EXAMPLES

The invention will be described in more detail with reference to the following Examples, which are not intended to restrict the scope thereof. In the Examples, "%" means "weight%" unless otherwise mentioned.

### Production (1) of monomer-A

### Synthesis of tris(trimethylsiloxy)silylnorbornene

A two-liter reactor was charged with 307 g of dicyclopetadiene and 750 g of tristrimethylsiloxyvinylsilane. Reaction was carried out at 160 to 170°C for eight hours, with the atmosphere sealed with nitrogen. The reaction solution was distilled under reduced pressure. There was obtained a fraction (757 g) having a boiling point of 118 to 120°C at 5 mmHg. This fraction was found to be a compound represented by the structural formula below as the result of ¹H-NMR analysis (with LAMBDA LA-300W made by JEOL Co., Ltd.) and IR analysis (with FT-IR Spectrometer Spectrum One made by Perkin Elmer Co., Ltd.). This compound is designated as monomer-A.

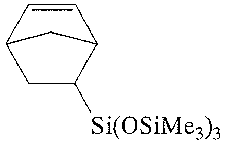

### Production (2) of monomer-B

### Synthesis of bis(trimethylsiloxy)methylsilylnorbornene

A 100-mL reactor was charged with 13 g of dicyclopentadiene and 50 g (0.20 mol) g of bistrimethylsiloxymethylvinylsilane. Reaction was carried out at 160 to 170° C for four hours under nitrogen stream. The reaction solution was distilled under reduced pressure. There was obtained a fraction (37 g) having a boiling point of 97 to 102° C at 9 mmHg and also having a refractive index (n_{D} 25) of 1.444. This fraction was found to be a compound represented by the structural formula below as the result of ¹H-NMR analysis (with LAMBDA LA-300W made by JEOL Co., Ltd.) and IR analysis (with FT-IR Spectrometer Spectrum One made by Perkin Elmer Co., Ltd.). This compound is designated as monomer-B.

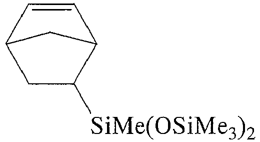

### Production (1) of polymer (I-1)

### Synthesis of tris(trimethylsiloxy)silylnorbornene-co-norbornene addition copolymer

A glass container with its atmosphere replaced by nitrogen was charged with 140 mL of toluene. In this toluene were dissolved 44.8 g (0.115 mol) of monomer-A, 5.8 g (0.062 mol) of monomer-C (norbornene), and 37 mg (40 µmol) of trityltetra(pentafluorophenyl)borate [Ph₃C][B(C₆F₅)₄].

To the container were further added a catalyst solution prepared separately including 9 mg (40 µmol) of cyclopentadienyl(aryl)palladium [C₅H₅PdC₃H₅] and 12 mg (40 µmol) of tricyclohexylphosphine (PCy₃), which are dissolved in 15 mL of toluene. Polymerization reaction was carried out at room temperature (25° C) for five hours.

After the completion of reaction, the reaction product was poured into a large amount of methanol so that the polymer separated out. After filtration and washing, the separated solids were dried under reduced pressure at 60° C for five hours. Thus there was obtained the desired polymer (I-1) in an amount of 31.8 g (yields: 63%).

The thus obtained polymer was found to have a molecular weight (Mn) of 601,000 (measured by GPC) and a molecular weight distribution (Mw/Mn) of 1.49. Analysis by ¹H-NMR spectrum indicated that the polymer is composed of monomer-A and monomer-C in a ratio of A/C = 67/33 (mol/mol).

### Production (2) of polymer (I-2)

### Synthesis of tris(trimethylsiloxy)silylnorbornene-co-norbornene addition copolymer (with a low molecular weight)

A glass container with its atmosphere replaced by nitrogen was charged with 400 mL of toluene. In this toluene were dissolved 202.4 g (0.52 mol) of monomer-A, 26.4 g (0.28 mol) of monomer-C (norbornene), and 1.1 g (0.01 mol) of 1-octene.

To the container were further added a catalyst solution prepared separately containing 6 mg (20 µmol) of bis(acetylacetonate)palladium [Pd(C₅H₇O₂)₂], 3 mg (10 µmol) of tricyclohexylphosphine (PCy₃), and 22 mg (24 µmol) of trityltetra(pentafluorophenyl)borate {[Ph₃C][B(C₆F₅)₄]}, which are dissolved in 15 mL of toluene. Polymerization reaction was carried out at 60° C for ten hours.

After the completion of reaction, the reaction product was poured into a large amount of methanol so that the polymer separated out. After filtration and washing, the separated solids were dried under reduced pressure at 120° C for eight hours. Thus there was obtained the desired polymer (I-2) in an amount of 160.2 g (yields: 70%).

The thus obtained polymer was found to have a molecular weight (Mn) of 230,000 (measured by GPC) and a molecular weight distribution (Mw/Mn) of 2.56. Analysis by ¹H-NMR spectrum indicated that the polymer is composed of monomer-A and monomer-C in a ratio of A/C = 67/33 (mol/mol).

### Production (3) of polymer (II)

### Synthesis of bis(trimethylsiloxy)methylsilylnorbornene-co-norbornene addition copolymer

The same procedure as in Production (1) of polymer was repeated except that monomer-A was replaced by 36.2 g (0.115 mol) of monomer-B. There was obtained the polymer (II) in an amount of 29.4 g (yields: 70%). This polymer was found to have a molecular weight (Mn) of 724,000 and a molecular weight distribution (Mw/Mn) of 1.38 and was also found to be composed of monomer-B and monomer-C in a ratio of 68/32 (mol/mol).

### Production (4) of polymer (III)

### Synthesis of bis(trimethylsiloxy)methylsilylnorbornene polymer

The same procedure as in Production (2) of polymer was repeated except that monomer-B alone was used in an amount of 55.7 g (0.177 mol). There was obtained the polymer (III) in an amount of 30.6 g (yields: 55%). This polymer was found to have a molecular weight (Mn) of 632,000 and a molecular weight distribution (Mw/Mn) of 1.39.

### Solution of polymer (I-1) in methyltrimethicone (M3T)

Polymer (I-1) and M3T were placed in a separable flask, with its atmosphere replaced by nitrogen. After dissolution at 80° C by a glass stirrer, there was obtained a 30 wt% homogeneous solution.

### Solution of polymer (I-1) in isododecane

The same procedure as above was repeated to give a 30% solution except that the solvent was replaced by isododecane. The resulting solution was found to have a viscosity of 700 Pa·s at 25° C.

For the purpose of comparison, polymer (I-1) and isododecane were mixed in a separable flask with its atmosphere replaced by nitrogen at 80° C by a glass stirrer to give a 30 wt% solution. However, the resulting product was found to lack fluidity at 25°C. The procedure was modified to give a 20 wt% solution and a 10 wt% solution. However, these solutions were found to have a viscosity of 1,500 Pa·s and 6.5 Pa·s, respectively.

### Solution of polymer (I-2) in dimethicone

Polymer (I-2) and dimethicone (KF-96L-2 cs, made by Shin-Etsu Chemical Co., Ltd.) were placed in a separable flask, with its atmosphere replaced by nitrogen. After dissolution at 80°C by a glass stirrer, there was obtained a 30 wt% homogeneous solution.

### Solution of polymer (I-2) in decamethylcyclopentasiloxane (D5)

Polymer (I-2) and D5 were placed in a separable flask, with its atmosphere replaced by nitrogen. After dissolution at 80°C by a glass stirrer, there was obtained a 10 wt% homogeneous solution.

### Solution of polymer (II) in M3T

Polymer (II) and M3T were placed in a separable flask, with its atmosphere replaced by nitrogen. After dissolution at 80° C by a glass stirrer, there was obtained a 10 wt% homogeneous solution.

### Solution of polymer (III) in D5

Polymer (III) and D5 were placed in a separable flask, with its atmosphere replaced by nitrogen. After dissolution at 80°C by a glass stirrer, there was obtained a 10% homogeneous solution.

Those polymers obtained as mentioned above are soluble in volatile solvents, such as isododecane, D5, dimethicone (2 cs), and M3T, which are used for cosmetic products. The resulting solutions vary in viscosity depending on the composition and molecular weight of the polymer. Consequently, the polymers of the present invention can be used as a thickener for cosmetic products. For example, the solution in D5 has a much higher viscosity than the solution in isododecane. Thus, the solution in D5 can be used to increase the viscosity of the solution of polymer (I-1) or (I-2) in isododecane.

As mentioned above, when dissolved in a volatile solvent, the polymer of the present invention gives a solution that forms a flexible tough film. Therefore, the cosmetic product incorporated with the solution of the polymer, even in small quantities, will exhibit improved rubbing resistance and transfer resistance. Moreover, the polymer of the present invention greatly shrinks when it forms film. Thus, it is expected to find use as antiwrinkle preparations and hair and eyelash preparations. In addition, the polymer (I-1), which has a high molecular weight, easily forms strings and becomes sticky when dried; therefore, it will be used for an improved mascara that forms thicker and longer eyelashes.

### Example 1 and Comparative Examples 1 and 2

Each example demonstrates the cream foundation of emulsion type having the composition shown in Table 1.

**Table 1**

| No. | Composition (weight%) | Example | Comparative Example | |
|---|---|---|---|---|
| | | 1 | 1 | 2 |
| 1 | Crosslinked polyether-modified silicone (*1) | 5.0 | 5.0 | 5.0 |
| 2 | Crosslinked dimethylpolysiloxane (*2) | 6.0 | 6.0 | 6.0 |
| 3 | Polyether-modified silicone (*3) | 1.0 | 1.0 | 1.0 |
| 4 | Dimethylpolysiloxane (*4) | 3.0 | 3.0 | 3.0 |
| 5 | Decamethylcyclopentasiloxane | 5.0 | 6.2 | 5.0 |
| 6 | Triethylhexanoin | 5.0 | 5.0 | 5.0 |
| 7 | Neopentylglycol dioctanoate | 2.0 | 2.0 | 2.0 |
| 8 | Polymethylsilsesquioxane powder (*5) | 1.5 | 1.5 | 1.5 |
| 9 | 1,3-BG | 5.0 | 5.0 | 5.0 |
| 10 | Sodium chloride | 0.5 | 0.5 | 0.5 |
| 11 | Water | 50.0 | 50.0 | 50.0 |
| 12 | Silicone-treated titanium oxide (*6) | 8.65 | 8.65 | 8.65 |
| 13 | Silicone-treated red iron oxide (*6) | 0.45 | 0.45 | 0.45 |
| 14 | Silicone-treated yellow iron oxide (*6) | 0.75 | 0.75 | 0.75 |
| 15 | Silicone-treated black iron oxide (*6) | 0.15 | 0.15 | 0.15 |
| 16 | 30 wt% solution of polymer (I-2) in dimethicone (2 cs) | 3.0 | -- | -- |
| 17 | Silicone resin (*7) | -- | 1.8 | -- |
| 18 | Silicone-modified acrylic polymer (*8) | -- | -- | 3.0 |
| 19 | Antioxidant | q.s. | q.s. | q.s. |
| 20 | Antiseptic | q.s. | q.s. | q.s. |
| 21 | Perfume | q.s | q.s. | q.s. |
| Total | | 100.0 | 100.0 | 100.0 |

| | | | | |
|---|---|---|---|---|
| *1: KSG-210 from Shin-Etsu Chemical Co., Ltd. *2: KSG-15 from Shin-Etsu Chemical Co., Ltd. *3: KF-6017 from Shin-Etsu Chemical Co., Ltd. *4: KF-96A-6 cs from Shin-Etsu Chemical Co., Ltd. *5: KMP-590 from Shin-Etsu Chemical Co., Ltd. *6: treated with KF-9909 from Shin-Etsu Chemical Co., Ltd. *7: KF-7312J (50 wt% solution of silicone resin in D5) from Shin-Etsu Chemical Co., Ltd. *8: KP-545 (30 wt% solution of silicone-modified acrylic polymer in D5) from Shin-Etsu Chemical Co., Ltd. | | | | |

### [Preparation of cosmetic product]

Ingredients (1) to (8), (19), and (20) were uniformly mixed by stirring. To the resulting mixture was slowly added for emulsification a homogenous solution of ingredients (9) and (10) dissolved in ingredient (11). The emulsified product was mixed with ingredients (12) to (18) and (21) which had been rolled previously. The resulting mixture was filled into prescribed containers. Thus there was obtained the cream foundation of emulsion type as desired. This cosmetic product was evaluated in the following way.

### [Evaluation of usability]

The cream foundation of emulsion type which was obtained as mentioned above was rated for usability by 50 female expert panelists according to the following criteria.

Items of usability rated: Ease of spreading, sticky feel, unevenness, transparency, and lasting quality (rated eight hours after application).

The results are shown below in terms of average.

### [Effect of preventing secondary sticking]

Each sample was applied in a similar manner to the forehead of the panelist, and, 20 minutes after application, a piece of tissue paper was pressed against the forehead to see if secondary sticking occurs. The sample was rated for the effect of preventing secondary sticking according to the following criteria. The results are shown below in terms of average.

**Table 2**

| Rating criteria | | | | | | |
|---|---|---|---|---|---|---|
| Point | Spreadability | Sticking | Unevenness | Transparency | Lasting | Secondary sticking |
| 5 | Good | None | None | Transparent | Good | None |
| 4 | Slightly good | Almost none | Almost none | Slightly transparent | Fair | Almost none |
| 3 | Moderate | Moderate | Moderate | Moderate | Moderate | Moderate |
| 2 | Slightly poor | Little | Little | Slightly opaque | Poor | Little |
| 1 | poor | Much | Much | Opaque | Bad | Much |

The results of rating are shown in Table 3 according to the average points.
⊚: average point higher than 4.0
○: average point from 3.0 to 4.0
×: average point from 2.0 to 3.0
××: average point lower than 2.0

**Table 3**

| Item | Example | Comparative Example | |
|---|---|---|---|
| | 1 | 1 | 2 |
| Spreading | ⊚ | ○ | ⊚ |
| Sticky feel | ⊚ | ○ | ⊚ |
| Unevenness | ⊚ | ○ | ○ |
| Transparency | ⊚ | ○ | ⊚ |
| Lasting quality | ⊚ | ×× | × |
| Secondary sticking | ⊚ | × | × |

It is apparent from Table 2 that the cosmetic product according to the present invention is by far superior to those in Comparative Examples 1 and 2 in lasting quality and secondary sticking as well as usability.

The following formulations were used to prepare various cosmetic products according to the present invention. The samples were rated for "spreading" and "lasting quality" in the same way as mentioned above. The foundation was also rated for secondary sticking in the same way as mentioned above.

### Example 2 - reference example

### Powder foundation

| | Formulation | weight % |
|---|---|---|
| 1. | Silicone-treated titanium oxide (*1) | 12.0 |
| 2. | Silicone-treated sericite (*1) | 35.0 |
| 3. | Lecithin-treated talc | 35.1 |
| 4. | Lecithin-treated spherical nylon powder | 5.0 |
| 5. | Silicone-treated red oxide (*1) | 0.4 |
| 6. | Silicone-treated yellow iron oxide (*1) | 2.0 |
| 7. | Silicone-treated amber (*1) | 0.4 |
| 8. | Silicone-treated black iron oxide (*1) | 0.1 |
| 9. | 10 wt% solution of polymer (I-2) in D5 | 3.0 |
| 10. | Crosslinked dimethylsiloxane (*2) | 4.0 |
| 11. | Glyceryl trioctanoate | 1.5 |
| 12. | Silicone wax (*3) | 1.5 |
| | Total | 100.0 |

| | | |
|---|---|---|
| *1: Treated with KF-9909 from Shin-Etsu Chemical Co., Ltd. *2: KSG-16 from Shin-Etsu Chemical Co., Ltd. *3: KP-562P from Shin-Etsu Chemical Co., Ltd. | | |

### <Preparation of cosmetic product>

A: prepared from ingredients 1 to 8 by uniform mixing and crushing.
B: prepared from ingredients 9 to 12 by uniform mixing.
C: prepared by adding B to A, followed by uniform mixing.

The resulting mixture was press-formed in a mold to give the powder foundation as desired. The thus obtained powder foundation was found to be good in spreading and lasting quality and free of secondary sticking.

### Example 3- reference example

### Powder foundation

| | Formulation | weight% |
|---|---|---|
| 1. | Caprylylsilane-treated mica (*1) | 40.0 |
| 2. | Silicone-treated talc (*2) | 24.6 |
| 3. | Silicone-treated titanium oxide (*2) | 10.0 |
| 4. | Silicone-treated fine powder of titanium oxide (*2) | 5.0 |
| 5. | Silicone-treated barium sulfate (*2) | 10.0 |
| 6. | Silicone-treated foundation pigment (*2) | q.s. |
| 7. | Phenyl-modified hybrid silicone composite powder (*3) | 2.0 |
| 8. | Polymethylsilsesquioxane powder (*4) | 0.4 |
| 9. | Antiseptic | q.s. |
| 10. | Perfume | q.s. |
| 11. | 10 wt% solution of polymer (III) in D5 | 4.0 |
| 12. | Glyceryl trioctanoate | 2.0 |
| 13. | Squalane | 1.0 |
| 14. | Vaseline | 1.0 |
| | Total | 100.0 |

| | | |
|---|---|---|
| *1: Treated with AES-3083 from Shin-Etsu Chemical Co., Ltd. *2: Treated with KF-9909 from Shin-Etsu Chemical Co., Ltd. *3: KSP-300 from Shin-Etsu Chemical Co., Ltd. *4: KMP-590 from Shin-Etsu Chemical Co., Ltd. | | |

### <Preparation of cosmetic product>

A: prepared from ingredients 1 to 9 by uniform mixing and crushing.
B: prepared from ingredients 11 to 14 by uniform mixing and added to A.
C: prepared adding ingredient 10 to B. The resulting mixture was press-formed in a mold to give the powder foundation as desired.

The thus obtained powder foundation was found to be good in spreading and lasting quality and free of secondary sticking.

### Example 4

### W/O foundation in stick form

| | Formulation | weight% |
|---|---|---|
| 1. | Seresin | 5.5 |
| 2. | Inulin stearate | 2.0 |
| 3. | Neopentylglycol dioctanoate | 8.0 |
| 4. | Triethylhexanoin | 4.0 |
| 5. | Dimethylpolysiloxane (6 cs) | 8.5 |
| 6. | 30 wt% solution of polymer (I-2) in dimethicone (2 cs) | 3.0 |
| 7. | Crosslinked polyglycerin-modified silicone (*1) | 4.0 |
| 8. | Alkyl-modified branched polyglycerin-modified silicone (*2) | 1.5 |
| 9. | Polymethylsilsesquioxane powder (*3) | 1.5 |
| 10. | Silicone-treated titanium oxide (*4) | 9.0 |
| 11. | Silicone-treated foundation pigment (*5) | q.s. |
| 12. | Lecithin | 0.2 |
| 13. | Polysorbate 80 | 0.3 |
| 14. | 1,3-BG | 5.0 |
| 15. | Antiseptic | q.s. |
| 16. | Perfume | q.s. |
| 17. | Purified water | 47.5 |
| | Total | 100.0 |

| | | |
|---|---|---|
| *1: KSG-710 from Shin-Etsu Chemical Co., Ltd. *2: KF-6105 from Shin-Etsu Chemical Co., Ltd. *3: KMP-590 from Shin-Etsu Chemical Co., Ltd. *4: treated with KF-9909 from Shin-Etsu Chemical Co., Ltd. *5: treated with KF-9909 from Shin-Etsu Chemical Co., Ltd. | | |

### <Preparation of cosmetic product>

A: prepared from ingredients 1 to 9 by uniform heating and dissolution.
B: prepared from ingredients 10 to 13 and a portion of ingredient 14 by rolling for dispersion.
C: prepared by uniformly dissolving the remainder of ingredient 14 and ingredients 15 and 17. C was added to B, followed by uniform dispersion with heating.
D: prepared by adding C to A for emulsification. After stirring with heating, ingredient 16 was added to D. The resulting product in stick form was filled into prescribed air-tight containers. Thus there was obtained the W/O foundation in stick form as desired.

The thus obtained W/O foundation was found to be good in spreading and lasting quality and free of secondary sticking.

### Example 5

### Oil-in-polyhydric alcohol emulsion cream

| | Formulation | weight% |
|---|---|---|
| 1. | Crosslinked polyglycerin-modified silicone (*1) | 5.0 |
| 2. | Crosslinked dimethylpolysiloxane (*2) | 30.0 |
| 3. | Crosslinked dimethylpolysiloxane (*3) | 35.0 |
| 4. | Branched polyglycerin-modified silicone (*4) | 1.0 |
| 5. | Decamethylcyclopentasiloxane | 4.0 |
| 6. | 10 wt% solution of polymer (III) in D5 | 5.0 |
| 7. | Phenyl-modified hybrid silicone composite powder (*5) | 5.0 |
| 8. | Antiseptic | q.s. |
| 9. | Perfume | q.s. |
| 10. | Glycerin | 5.0 |
| 11. | 1,3-butylene glycol | 10.0 |
| | Total | 100.0 |

| | | |
|---|---|---|
| *1: KSG-710 from Shin-Etsu Chemical Co., Ltd. *2: KSG-15 from Shin-Etsu Chemical Co., Ltd. *3: KSG-1610 from Shin-Etsu Chemical Co., Ltd. *4: KF-6104 from Shin-Etsu Chemical Co., Ltd. *5: KSP-300 from Shin-Etsu Chemical Co., Ltd. | | |

### <Preparation of cosmetic product>

A: prepared from ingredients 1 to 7 and 9 by uniform mixing.
B: prepared from ingredients 8, 10, and 11 by mixing.
C: prepared by adding B to A, followed by uniform emulsification. There was obtained the cream as desired.

The oil-in-polyhydric alcohol emulsion cream obtained as mentioned above was found to be good in spreading and moist feel and free of sticky feel and oily feel.

### Example 6

### Blusher in solid form of oil-in-polyhydric alcohol emulsion type

| | Formulation | weight% |
|---|---|---|
| 1. | Crosslinked polyglycerin-modified silicone (*1) | 5.0 |
| 2. | Crosslinked dimethylpolysiloxane (*2) | 5.0 |
| 3. | Decamethylcyclopentasiloxane | 3.0 |
| 4. | Dimethylpolysiloxane (6 cs) | 19.7 |
| 5. | Cetyl isooctanoate | 5.0 |
| 6. | 10 wt% solution of polymer (III) in D5 | 10.0 |
| 7. | Behenyl-modified acrylsilicone resin (*3) | 3.0 |
| 8. | Paraffin wax (m.p. 80° C) | 9.0 |
| 9. | Dimethyldistearylammoniumhectorite | 0.3 |
| 10. | Acrylsilicone-treated powder (*4) | 25.0 |
| 11. | Antiseptic | q.s. |
| 12. | Perfume | q.s. |
| 13. | 1,3-butylene glycol | 15.0 |
| | Total | 100.0 |

| | | |
|---|---|---|
| *1: KSG-710 from Shin-Etsu Chemical Co., Ltd. *2: KSG-15 from Shin-Etsu Chemical Co., Ltd. *3: KP-562P from Shin-Etsu Chemical Co., Ltd. *4: treated with KP-574 from Shin-Etsu Chemical Co., Ltd. | | |

### <Preparation of cosmetic product>

A: prepared from ingredients 1 to 9 and 12 by uniform mixing with heating at 80° C.
B: prepared by adding ingredient 10 to A, followed by uniform dispersion.
C: prepared from ingredients 11 and 13 by mixing. After heating to 80° C, C was added to B for emulsification. The resulting emulsion was cast into a metal dish, followed by cooling. Thus there was obtained the blusher as desired.

The blusher in solid form of oil-in-polyhydric alcohol emulsion type obtained as mentioned above was found to be good in spreading and free of sticky feel and oily feel.

### Example 7

### Lip color in cream form

| | Formulation | weight% |
|---|---|---|
| 1. | Dextrin palmitate/ethylhexanoate (*1) | 9.0 |
| 2. | Triethylhexanoin | 10.0 |
| 3. | 30 wt% solution of polymer (I-1) in M3T | 8.0 |
| 4. | Alkyl-modified crosslinked dimethyl-polysiloxane (*2) | 8.0 |
| 5. | Alkyl-modified branched polyglycerin-modified silicone (*3) | 2.0 |
| 6. | Decamethylcyclopentasiloxane | 40.0 |
| 7. | 1,3-butylene glycol | 5.0 |
| 8. | Purified water | 18.0 |
| 9. | Colored pigment | q.s. |
| 10. | Mica | q.s. |
| 11. | Perfume | q.s. |
| | Total | 100.0 |

| | | |
|---|---|---|
| *1: Rheopearl TT from Chiba Flour Milling Co., Ltd. *2: KSG-43 from Shin-Etsu Chemical Co., Ltd. *3: KF-6105 from Shin-Etsu Chemical Co., Ltd. | | |

### <Preparation of cosmetic product>

A: prepared from a portion of ingredients 1 and 2 and ingredients 3 to 6 by uniform mixing with heating.
B: prepared by adding ingredient 9 to the remainder of ingredient 2 by rolling for dispersion. B was added to A, followed by uniform mixing.
C: prepared from ingredients 7 and 8 by mixing with heating. C was added to B with heating, followed by emulsification.
D: prepared by adding ingredients 10 and 11 to C. Thus there was obtained the lip color in cream form as desired.

The lip color in cream form obtained as mentioned above was found to be good in spreading on lips and lasting quality and free of oily feel.

### Example 8

### Eye liner

| | Formulation | weight% |
|---|---|---|
| 1. | Methyl trimethicone (*1) | 20.0 |
| 2. | Polyether-modified silicone (*2) | 3.0 |
| 3. | 30 wt% solution of polymer (I-1) in M3T | 33.5 |
| 4. | Solution of silicone network resin (*3) | 15.0 |
| 5. | Dimethyldistearylammoniumhectorite | 3.0 |
| 6. | Silicone-modified black iron oxide (*4) | 10.0 |
| 7. | 1,3-butylene glycol | 5.0 |
| 8. | Sodium sulfate | 0.5 |
| 9. | Antiseptic | q.s. |
| 10. | Purified water | 10.0 |
| | Total | 100.0 |

| | | |
|---|---|---|
| *1: TMF-1.5 from Shin-Etsu Chemical Co., Ltd. *2: KF-6017 from Shin-Etsu Chemical Co., Ltd. *3: KF-7312T from Shin-Etsu Chemical Co., Ltd. *4: treated with KF-9901 from Shin-Etsu Chemical Co., Ltd. | | |

### <Preparation of cosmetic product>

A: prepared from ingredients 1 to 5 by mixing, and ingredient 6 was added to the mixture by uniform mixing for dispersion.
B: prepared from ingredients 7 to 10 by mixing.
C: prepared by adding B to A, followed by emulsification. Thus there was obtained the eye liner as desired.

The eye liner obtained as mentioned above was found to be easy to use with refreshed feel and very good in lasting quality.

Although M3T was used as the volatile oil in the formulation, it may be replaced by isododecane or solution of polymer (I-1) in isododecane (30 wt%).

### Example 9⁻ reference example

### Mascara

| | Formulation | weight% |
|---|---|---|
| 1. | 30 wt% solution of polymer (I-1) in isododecane | 26.5 |
| 2. | Dextrin palmitate/ethylhexanoate (*1) | 3.0 |
| 3. | Seresin | 2.5 |
| 4. | Behenyl-modified acrylsilicone resin (*2) | 2.0 |
| 5. | Bees wax | 3.5 |
| 6. | Triethylhexanoin | 3.0 |
| 7. | Dimethyldistearylammoniumhectorite | 4.0 |
| 8. | Lecithin | 0.5 |
| 9. | Isododecane | 34.0 |
| 10. | Silicone-treated pigment (*3) | 5.0 |
| 11. | Silica | 3.0 |
| 12. | Talc | 12.0 |
| 13. | Branched polyether-modified silicone (*4) | 1.0 |
| Total | | 100.0 |

| | | |
|---|---|---|
| *1: Rheopearl TT from Chiba Flour Milling Co., Ltd. *2: KP-562P from Shin-Etsu Chemical Co., Ltd. *3: treated with KF-9909 from Shin-Etsu Chemical Co., Ltd. *4: KF-6028P from Shin-Etsu Chemical Co., Ltd. | | |

### <Preparation of cosmetic product>

A: prepared by adding ingredients 7 and 13 to ingredient 9, followed by uniform mixing with heating.
B: prepared by adding ingredients 1 to 6 and 8 to A, followed by uniform mixing.
C: prepared by adding ingredients 10 to 12 to B, followed by rolling for uniform mixing. Thus there was obtained the mascara as desired.

The mascara obtained as mentioned above was found to easily attach to eyelashes and be very good in spreading and lasting quality without sticky feel.

For comparison, a sample of mascara was prepared in the same way as above except that the solution of polymer (I-1) in isododecane was replaced by the polymer disclosed in JP-A 2010-024154, which was produced from the same monomer by ring-opening metathesis polymerization. The resulting sample gave a sticky and uncomfortable feeling.

### Example 10

### Cream eye shadow

| | Formulation | weight% |
|---|---|---|
| 1. | Decamethylcyclopentasiloxane | 15.0 |
| 2. | Dimethylpolysiloxane (6 cs) | 4.0 |
| 3. | 30 wt% solution of polymer (I-2) in dimethicone (2 cs) | 5.0 |
| 4. | Branched polyether-modified silicone (*1) | 1.5 |
| 5. | Pigment treated with acrylsilicone resin (*2) | 16.0 |
| 6. | Sodium chloride | 2.0 |
| 7. | Propylene glycol | 8.0 |
| 8. | Antiseptic | q.s. |
| 9. | Purified water | 48.5 |
| Total | | 100.0 |

| | | |
|---|---|---|
| *1: KF-6028P from Shin-Etsu Chemical Co., Ltd. *2: treated with KP-574 from Shin-Etsu Chemical Co., Ltd. | | |

### <Preparation of cosmetic product>

A: prepared from ingredients 1 to 4 by mixing, and then ingredient 5 was added, followed by uniform mixing for dispersion.
B: prepared from ingredients 6 to 9 by mixing.
C: prepared by adding B to A. Thus there was obtained the cream eye shadow as desired.

The cream eye shadow obtained as mentioned above was found to spread easily and last long without oily feeling and powdery feeling.

### Example 11

### Cream eye shadow

| | Formulation | weight% |
|---|---|---|
| 1. | Solution of acrylsilicone resin (*1) | 3.0 |
| 2. | Stearyl-modified acrylsilicone resin (*2) | 2.0 |
| 3. | Branched polyether-modified silicone (*3) | 1.5 |
| 4. | Decamethylcyclopentasiloxane | 20.3 |
| 5. | 30 wt% solution of polymer (I-1) in M3T | 10.0 |
| 6. | Dimethyldistearylammoniumhectorite | 1.2 |
| 7. | Pigment treated with acrylsilicone resin (*4) | 20.0 |
| 8. | Spherical nylon powder | 3.0 |
| 9. | Talc | 4.0 |
| 10. | Ethanol | 5.0 |
| 11. | Purified water | 30.0 |
| Total | | 100.0 |

| | | |
|---|---|---|
| *1: KP-545 from Shin-Etsu Chemical Co., Ltd. *2: KP-561P from Shin-Etsu Chemical Co., Ltd. *3: KF-6028P from Shin-Etsu Chemical Co., Ltd. *4: treated with KP-574 from Shin-Etsu Chemical Co., Ltd. | | |

### <Preparation of cosmetic product>

A: prepared from ingredients 1 to 6 by mixing, and then ingredients 7 to 9 were added, followed by uniform mixing for dispersion.
B: prepared from ingredients 10 and 11 by mixing.
C: prepared by adding B to A. Thus there was obtained the cream eye shadow as desired.

The cream eye shadow obtained as mentioned above was found to spread easily, last long, and remain fresh without oily feeling and powdery feeling.

### Example 12

### Suncut milky lotion

| | Formulation | weight% |
|---|---|---|
| 1. | Crosslinked polyether-modified silicone (*1) | 3.0 |
| 2. | Crosslinked dimethylpolysiloxane (*2) | 2.0 |
| 3. | Branched polyether-modified silicone (*3) | 1.0 |
| 4. | 30 wt% solution of polymer (I-1) in isodecane | 5.0 |
| 5. | Decamethylcyclopentasiloxane | 5.0 |
| 6. | Isotridecyl isonanoate | 4.0 |
| 7. | Dispersion of titanium oxide (*4) | 25.0 |
| 8. | Dispersion of zinc oxide (*5) | 35.0 |
| 9. | 1,3-butylene glycol | 2.0 |
| 10. | Sodium citrate | 0.2 |
| 11. | Sodium chloride | 0.5 |
| 12. | Purified water | 17.3 |
| Total | | 100.0 |

| | | |
|---|---|---|
| *1: KSG-210 from Shin-Etsu Chemical Co., Ltd. *2: KSG-15 from Shin-Etsu Chemical Co., Ltd. *3: KF-6028P from Shin-Etsu Chemical Co., Ltd. *4: SPD-T5 from Shin-Etsu Chemical Co., Ltd. *5: SPD-Z5 from Shin-Etsu Chemical Co., Ltd. | | |

### <Preparation of cosmetic product>

A: prepared from ingredients 1 to 6 by mixing.
B: prepared from ingredients 9 to 12 by mixing.
C: prepared by adding B to A, followed by emulsification, and then ingredients 7 and 8 were added. Thus there was obtained the suncut milky lotion as desired.

The suncut milky lotion obtained as mentioned above was found to spread easily, and exhibit good sweat resistance without sticky feeling and oily feeling.

### Example 13

### Suncut cream

| | Formulation | weight% |
|---|---|---|
| 1. | Crosslinked polyether-modified silicone (*1) | 3.0 |
| 2. | Crosslinked dimethylpolysiloxane (*2) | 2.0 |
| 3. | Alkyl-modified branched polyether-modified silicone (*3) | 1.0 |
| 4. | 10 wt% solution of polymer (I-2) in D5 | 7.0 |
| 5. | Decamethylcyclopentasiloxane | 15.5 |
| 6. | Octyl methoxycinnamate | 6.0 |
| 7. | Solution of acrylsilicone resin (*4) | 10.0 |
| 8. | Lipophilized fine powder of zinc oxide (*5) | 20.0 |
| 9. | 1,3-butylene glycol | 2.0 |
| 10. | Sodium citrate | 0.2 |
| 11. | Sodium chloride | 0.5 |
| 12. | Perfume | q.s. |
| 13. | Purified water | 32.8 |
| Total | | 100.0 |

| | | |
|---|---|---|
| *1: KSG-240 from Shin-Etsu Chemical Co., Ltd. *2: KSG-15 from Shin-Etsu Chemical Co., Ltd. *3: KF-6038 from Shin-Etsu Chemical Co., Ltd. *4: KP-575 from Shin-Etsu Chemical Co., Ltd. *5: treated with AES-3083 from Shin-Etsu Chemical Co., Ltd. | | |

### <Preparation of cosmetic product>

A: prepared by adding ingredient 7 to a portion of ingredient 5 and adding ingredient 8, followed by dispersion in a beads mill.
B: prepared from ingredients 1 to 4, the remainder of ingredient 5, and ingredient 6 by uniform mixing.
C: prepared from ingredients 9 to 11 and 13 by uniform mixing.
D: prepared by adding C to B, followed by emulsification, and then A and ingredient 12 were added. Thus there was obtained the suncut cream as desired.

The suncut cream obtained as mentioned above was found to spread easily, last long, and exhibit good usability without sticky feeling and oily feeling.

### Example 14

### Suncut lotion (shaking type)

| | Formulation | weight% |
|---|---|---|
| 1. | Branched polyether-modified silicone (*1) | 2.0 |
| 2. | 30 wt% solution of polymer (I-1) in dimethicone (2 cs) | 5.0 |
| 3. | Dimethylpolysiloxane (6 cs) | 3.0 |
| 4. | Decamethylcyclopentasiloxane | 7.8 |
| 5. | Ethylhexyl methoxycinnamate | 7.5 |
| 6. | Hybrid silicone composite powder (*2) | 0.5 |
| 7. | Dimethyldistearylammoniumhectorite | 0.2 |
| 8. | Zinc oxide dispersion (*3) | 45.0 |
| 9. | 1,3-butylene glycol | 3.0 |
| 10. | Alcohol | 5.0 |
| 11. | Sodium citrate | 0.2 |
| 12. | Sodium chloride | 0.5 |
| 13. | Purified water | 20.3 |
| Total | | 100.0 |

| | | |
|---|---|---|
| *1: KF-6028P from Shin-Etsu Chemical Co., Ltd. *2: KSP-105 from Shin-Etsu Chemical Co., Ltd. *3: SPD-Z6 from Shin-Etsu Chemical Co., Ltd. | | |

### <Preparation of cosmetic product>

A: prepared from ingredients 1 to 7 by uniform mixing.
B: prepared from ingredients 9 to 13 by mixing.
C: prepared by adding B to A, followed by emulsification, and then ingredient 8 was added. Thus there was obtained the suncut lotion of shaking type as desired.

The suncut lotion obtained as mentioned above was found to spread easily and last long without sticky feeling and oily feeling.

### Example 15

### Suncut cream

| | Formulation | weight% |
|---|---|---|
| 1. | Crosslinked polyether-modified silicone (*1) | 2.0 |
| 2. | Crosslinked dimethylpolysiloxane (*2) | 3.0 |
| 3. | Branched polyether-modified silicone (*3) | 1.5 |
| 4. | 10 wt% solution of polymer (I-2) in D5 | 4.5 |
| 5. | Decamethylcyclopentasiloxane | 5.8 |
| 6. | Dimethyldistearylammoniumhectorite | 1.2 |
| 7. | Titanium oxide dispersion (*4) | 20.0 |
| 8. | Zinc oxide dispersion (*5) | 15.0 |
| 9. | 1,3-butylene glycol | 5.0 |
| 10. | Sodium citrate | 0.2 |
| 11. | Sodium chloride | 0.5 |
| 12. | Purified water | 41.3 |
| Total | | 100.0 |

| | | |
|---|---|---|
| *1: KSG-210 from Shin-Etsu Chemical Co., Ltd. *2: KSG-15 from Shin-Etsu Chemical Co., Ltd. *3: KF-6028P from Shin-Etsu Chemical Co., Ltd. *4: SPD-T6 from Shin-Etsu Chemical Co., Ltd. *5: SPD-Z6 from Shin-Etsu Chemical Co., Ltd. | | |

### <Preparation of cosmetic product>

A: prepared from ingredients 1 to 8 by uniform mixing.
B: prepared from ingredients 9 to 12 by mixing.
C: prepared by adding B to A. Thus there was obtained the suncut cream as desired.

The suncut cream obtained as mentioned above was found to spread easily and last long without sticky feeling and oily feeling.

### Example 16

### Suntan milky lotion

| | Formulation | weight% |
|---|---|---|
| 1. | Crosslinked polyether-modified silicone (*1) | 2.0 |
| 2. | Crosslinked dimethylpolysiloxane (*2) | 3.0 |
| 3. | Polyether-modified silicone (*3) | 1.5 |
| 4. | 10 wt% solution of polymer (III) in D5 | 10.0 |
| 5. | Dimethylpolysiloxane (6 cs) | 10.0 |
| 6. | Decamethylcyclopentasiloxane | 16.5 |
| 7. | Dihydroxyacetone | 2.0 |
| 8. | Glycerin | 8.0 |
| 9. | 1,3-butylene glycol | 5.0 |
| 10. | Sodium citrate | 0.2 |
| 11. | Sodium chloride | 0.5 |
| 12. | Antioxidant | q.s. |
| 13. | Antiseptic | q.s. |
| 14. | Perfume | q.s. |
| 15. | Purified water | 41.3 |
| Total | | 100.0 |

| | | |
|---|---|---|
| *1: KSG-210 from Shin-Etsu Chemical Co., Ltd. *2: KSG-15 from Shin-Etsu Chemical Co., Ltd. *3: KF-6017 from Shin-Etsu Chemical Co., Ltd. | | |

### <Preparation of cosmetic product>

A: prepared from ingredients 1 to 6 by uniform mixing.
B: prepared from ingredients 7 to 13 and 15 by mixing.
C: prepared by adding B to A, followed by addition of ingredient 14. Thus there was obtained the suntan cream as desired.

The suntan cream obtained as mentioned above was found to spread easily and give light feeling without sticky feeling and oily feeling.

### Example 17

### Suntan cream

| | Formulation | weight% |
|---|---|---|
| 1. | Alkyl-modified crosslinked polyether-modified silicone (*1) | 4.0 |
| 2. | Alkyl-modified crosslinked dimethylpoly-siloxane (*2) | 2.0 |
| 3. | Alkyl-modified branched polyether-modified silicone (*3) | 1.0 |
| 4. | 10 wt% solution of polymer (I-2) in D5 | 5.0 |
| 5. | Decamethylcyclopentasiloxane | 10.5 |
| 6. | Stearyl-modified acrylsilicone (*4) | 1.0 |
| 7. | Dimethyloctylparaaminobenzoic acid | 1.5 |
| 8. | 4-t-butyl-4'-methoxy-dibenzoylmethane | 1.5 |
| 9. | Kaolin | 0.5 |
| 10. | Pigment | 8.0 |
| 11. | Titanium oxide-coated mica | 8.0 |
| 12. | Dioctadecyldimethylammonium chloride | 0.1 |
| 13. | Sodium L-glutamate | 3.0 |
| 14. | 1,3-butylene glycol | 5.0 |
| 15. | Sodium citrate | 0.2 |
| 16. | Sodium chloride | 0.5 |
| 17. | Antioxidant | q.s. |
| 18. | Antiseptic | q.s. |
| 19. | Perfume | q.s. |
| 20. | Purified water | 48.2 |
| Total | | 100.0 |

| | | |
|---|---|---|
| *1: KSG-320 from Shin-Etsu Chemical Co., Ltd. *2: KSG-42 from Shin-Etsu Chemical Co., Ltd. *3: KF-6038 from Shin-Etsu Chemical Co., Ltd. *4: KP-561P from Shin-Etsu Chemical Co., Ltd. | | |

### <Preparation of cosmetic product>

A: prepared from ingredients 1 to 8, 17, and 18 by mixing with heating.
B: prepared from ingredients 12 and a portion of ingredient 20 by mixing with heating, and then ingredients 9 to 11 were added, followed by dispersion.
C: prepared from ingredients 13 to 16 and the remainder of ingredient 20 by uniform dissolution. Then, C was mixed with B.
D: prepared by slowly adding C to A with stirring for emulsification. After cooling, ingredient 19 was added. Thus there was obtained the suntan cream as desired.

The suntan cream obtained as mentioned above was found to have fine texture, spread easily, last long, and give light feeling without sticky feeling and oily feeling.

### Example 18

### Liquid W/O foundation

| | Formulation | weight% |
|---|---|---|
| 1. | Decamethylcyclopentasiloxane | 16.0 |
| 2. | Dimethylpolysiloxane (6 cs) | 2.0 |
| 3. | 30 wt% solution of polymer (I-1) in isododecane | 4.0 |
| 4. | 10 wt% solution of polymer (II) in M3T | 5.0 |
| 5. | Alkyl-modified branched polyether-modified silicone (*1) | 2.0 |
| 6. | Ethylhexyl paramethoxycinnamate | 3.0 |
| 7. | Fluorine-modified silicone (*2) | 2.0 |
| 8. | Polymethylsilsesquioxane powder (*3) | 1.5 |
| 9. | Foundation pigment treated with fluorine compound (*4) | 9.3 |
| 10. | Fine particles of mica titanium treated with silicone (*4) | 2.0 |
| 11. | Fine particles of titanium oxide treated with silicone (*5) | 8.0 |
| 12. | Alkyl-modified branched polyglycerin-modified silicone (*6) | 1.2 |
| 13. | Ethanol | 3.0 |
| 14. | 1,3-butylene glycol | 5.0 |
| 15. | Glycerin | 2.0 |
| 16. | Magnesium sulfate | 0.5 |
| 17. | Antioxidant | q.s. |
| 18. | Antiseptic | q.s. |
| 19. | Perfume | q.s. |
| 20. | Purified water | 33.5 |
| Total | | 100.0 |

| | | |
|---|---|---|
| *1: KF-6038 from Shin-Etsu Chemical Co., Ltd. *2: FL-5 from Shin-Etsu Chemical Co., Ltd. *3: KMP-590 from Shin-Etsu Chemical Co., Ltd. *4: coated with diethanolamine salt of perfluoroalkyl-ethylphosphate (5%) *5: treated with KF-9909 from Shin-Etsu Chemical Co., Ltd. *6: KF-6105 from Shin-Etsu Chemical Co., Ltd. | | |

### <Preparation of cosmetic product>

A: prepared from a portion of ingredient 1, ingredients 11 and 12, and a portion of ingredient 20 by mixing, followed by uniform dispersion.
B: prepared from ingredients 8 to 10 by uniform mixing.
C: prepared from the remainder of ingredient 1 and ingredients 2 to 7 by mixing. C was added to B for uniform dispersion and mixing.
D: prepared from ingredients 13 to 18 and the remainder of ingredient 20 by uniform mixing.
E: D was slowly added to C with stirring for emulsification, and A and ingredient 19 were added. Thus there was obtained the W/O foundation as desired.

The W/O foundation obtained as mentioned above was found to spread easily, last long, and give light feeling without sticky feeling and oily feeling and secondary sticking.

### Example 19

### Hair cream

| | Formulation | weight% |
|---|---|---|
| 1. | Decamethylcyclopentasiloxane | 16.0 |
| 2. | Methylphenylpolysiloxane (*1) | 2.0 |
| 3. | 10 wt% solution of polymer (I-2) in D5 | 4.0 |
| 4. | Squalane | 5.0 |
| 5. | Solution of silicone network resin (*2) | 2.0 |
| 6. | Sorbitan sesquiisostearate | 1.5 |
| 7. | Alkyl-modified branched polyether-modified silicone (*3) | 2.0 |
| 8. | Sodium sorbitol sulfate | 2.0 |
| 9. | Sodium chondroitin sulfate | 1.0 |
| 10. | Sodium hyaluronate | 0.5 |
| 11. | Propylene glycol | 3.0 |
| 12. | Antiseptic | 1.5 |
| 13. | Vitamin E acetate | 0.1 |
| 14. | Antioxidant | q.s. |
| 15. | Perfume | q.s. |
| 16. | Purified water | 59.4 |
| Total | | 100.0 |

| | | |
|---|---|---|
| *1: KF-54 from Shin-Etsu Chemical Co., Ltd. *2: KF-7312J from Shin-Etsu Chemical Co., Ltd. *3: KF-6038 from Shin-Etsu Chemical Co., Ltd. | | |

### <Preparation of cosmetic product>

A: prepared from ingredients 1 to 7 and 12 to 14 by uniform mixing.
B: prepared from ingredients 8 to 11 and 16 by uniform mixing.
C: prepared by slowly adding B to A with stirring for emulsification, and then ingredient 15 was added. Thus there was obtained the hair cream as desired.

The hair cream obtained as mentioned above was found to spread easily, last long, and exhibit good water resistance, water repellency, and sweat resistance without oily feeling.

### Example 20

### Hair cream

| | Formulation | weight% |
|---|---|---|
| 1. | Solution of silicone gum (*1) | 10.0 |
| 2. | Solution of silicone network resin (*2) | 10.0 |
| 3. | 10 wt% solution of polymer (II) in M3T | 10.0 |
| 4. | Glyceryl tri-2-ethylhexanoate | 5.0 |
| 5. | Vaseline | 5.0 |
| 6. | Stearic acid | 1.5 |
| 7. | Cetyl alcohol | 0.5 |
| 8. | Polyglyceryl monooleate | 1.5 |
| 9. | Glyceryl monostearate | 1.5 |
| 10. | Polyether-modified silicone (*3) | 0.5 |
| 11. | 1,3-butylene glycol | 5.0 |
| 12. | Acrylates/alkyl acrylate (C10-30) cross-polymer (*4) | 0.3 |
| 13. | Triethanolamine | 0.3 |
| 14. | Antiseptic | q.s. |
| 15. | Perfume | q.s. |
| 16. | Purified water | 48.9 |
| Total | | 100.0 |

| | | |
|---|---|---|
| *1: KF-9028 from Shin-Etsu Chemical Co., Ltd. *2: KF-7312J from Shin-Etsu Chemical Co., Ltd. *3: KF-6011 from Shin-Etsu Chemical Co., Ltd. *4: Pemulen TR-1 from Noveon Inc. | | |

### <Preparation of cosmetic product>

A: prepared from ingredients 1 to 10 and 14 by heating for dissolution.
B: prepared from ingredients 11 to 13 and 16 by mixing with heating.
C: prepared by adding B to A with stirring for emulsification. After cooling, ingredient 15 was added. Thus there was obtained the hair cream as desired.

The hair cream obtained as mentioned above was found to spread easily, last long, impart gloss and smoothness to hair, produce good hair setting effect, and exhibit good water resistance and sweat resistance.

### Example 21

### Moisturizing O/W cream

| | Formulation | weight% |
|---|---|---|
| 1. | Decamethylcyclopentasiloxane | 7.0 |
| 2. | 10 wt% solution of polymer (I-2) in D5 | 6.0 |
| 3. | Liquid paraffin | 5.0 |
| 4. | Stearoxy-modified silicone (*1) | 8.0 |
| 5. | Polyether-modified silicone (*2) | 2.0 |
| 6. | Hybrid silicone composite powder (*3) | 2.5 |
| 7. | Hydrophobic silica | 2.0 |
| 8. | Zinc stearate | 2.0 |
| 9. | Vitamin E acetate | 3.0 |
| 10. | Polyacrylamide (*4) | 0.5 |
| 11. | Polyethylene glycol 400 | 1.0 |
| 12. | Sodium lactate | 1.0 |
| 13. | 1,3-butylene glycol | 5.0 |
| 14. | Antiseptic | q.s. |
| 15. | Perfume | q.s. |
| 16. | Purified water | 55.0 |
| Total | | 100.0 |

| | | |
|---|---|---|
| *1: KF-7002 from Shin-Etsu Chemical Co., Ltd. *2: KF-6011P from Shin-Etsu Chemical Co., Ltd. *3: KSP-100 from Shin-Etsu Chemical Co., Ltd. *4: Sepigel 305 from Sepik Co., Ltd. | | |

### <Preparation of cosmetic product>

A: prepared from ingredients 1 to 9 and 14 by uniform mixing.
B: prepared from ingredients 10 to 13 and 16 by uniform mixing.
C: prepared by adding B to A with stirring for emulsification, then ingredient 15 was added. Thus there was obtained the moisturizing O/W cream as desired.

The moisturizing O/W cream obtained as mentioned above was found to spread easily, impart refreshing feeling, and keep long the moisturizing effect.

### Example 22

### O/W emollient cream

| | Formulation | weight% |
|---|---|---|
| 1. | Crosslinked dimethylpolysiloxane (*1) | 7.0 |
| 2. | Crosslinked dimethylpolysiloxane (*2) | 30.0 |
| 3. | 10 wt% solution of polymer (I-2) in D5 | 6.0 |
| 4. | Decamethylcyclopentasiloxane | 5.0 |
| 5. | 1,3-butylene glycol | 4.0 |
| 6. | Branched polyglycerin-modified silicone (*3) | 0.6 |
| 7. | Branched polyglycerin-modified silicone (*4) | 0.3 |
| 8. | (Acrylamide/acryloyldimethyltaulin Na) copolymer (*5) | 0.6 |
| 9. | Dimethyltaulinammonium acrylate/VP copolymer (*6) | 0.7 |
| 10. | Sodium chloride | 0.1 |
| 11. | Purified water | 45.7 |
| Total | | 100.0 |

| | | |
|---|---|---|
| *1: KSG-15 from Shin-Etsu Chemical Co., Ltd. *2: KSG-16 from Shin-Etsu Chemical Co., Ltd. *3: KF-6104 from Shin-Etsu Chemical Co., Ltd. *4: KF-6100 from Shin-Etsu Chemical Co., Ltd. *5: Simulgel 600 from Sepik Co., Ltd. *6: Aristoflex AVC from Clariant Inc. | | |

### <Preparation of cosmetic product>

A: prepared from ingredients 1 to 4 by uniform mixing.
B: prepared from ingredients 5 to 11 by uniform mixing.
C: prepared by adding B to A with stirring. Thus there was obtained the O/W emollient cream as desired.

The O/W emollient cream obtained as mentioned above was found to spread easily, impart dry feeling, and effectively protect the skin without oily feeling.

### Example 23

### Hand cream

| | Formulation | weight% |
|---|---|---|
| 1. | Decamethylcyclopentasiloxane | 25.0 |
| 2. | 10 wt% solution of polymer (III) in D5 | 10.0 |
| 3. | Liquid paraffin | 5.0 |
| 4. | Solution of amino-modified silicone gum (*1) | 8.0 |
| 5. | Branched polyether-modified silicone (*2) | 2.0 |
| 6. | Hybrid silicone composite powder (*3) | 2.5 |
| 7. | Distearyldimethylammonium chloride | 0.8 |
| 8. | Vitamin E acetate | 0.1 |
| 9. | Polyethylene glycol 400 | 1.0 |
| 10. | Glycerin | 10.0 |
| 11. | Aluminum magnesium silicate | 1.2 |
| 12. | Antiseptic | q.s. |
| 13. | Perfume | q.s. |
| 14. | Purified water | 34.4 |
| Total | | 100.0 |

| | | |
|---|---|---|
| *1: KF-8108 from Shin-Etsu Chemical Co., Ltd. *2: KF-6028P from Shin-Etsu Chemical Co., Ltd. *3: KSP-102 from Shin-Etsu Chemical Co., Ltd. | | |

### <Preparation of cosmetic product>

A: prepared from ingredients 1 to 8 and 12 by uniform mixing.
B: prepared from ingredients 9 to 11 and 14 by uniform mixing.
C: prepared by adding B to A with stirring for emulsification, and then ingredient 13 was added.
   Thus there was obtained the hand cream as desired.

The hand cream obtained as mentioned above was found to spread easily, impart dry feeling, and effectively protect the skin without oily feeling.

### Example 24

### O/W cream

| | Formulation | weight% |
|---|---|---|
| 1. | Dimethylpolysiloxane (6 cs) | 5.0 |
| 2. | Stearyl-modified acrylsilicone resin (*1) | 8.0 |
| 3. | 10 wt% solution of polymer (I-2) in D5 | 10.0 |
| 4. | Glyceryl triisostearate | 8.0 |
| 5. | Cetanol | 1.0 |
| 6. | Stearic acid | 3.0 |
| 7. | Glyceryl monostearate | 1.5 |
| 8. | Sorbitan sesquioleate | 0.5 |
| 9. | Polyoxyethylenesorbitan monooleate | 1.0 |
| 10. | Sodium hydroxide (1 wt% aqueous solution) | 10.0 |
| 11. | 1,3-butylene glycol | 5.0 |
| 12. | Antiseptic | q.s. |
| 13. | Perfume | q.s. |
| 14. | Purified water | 54.3 |
| Total | | 100.0 |

| | | |
|---|---|---|
| *1: KP-561P from Shin-Etsu Chemical Co., Ltd. | | |

### <Preparation of cosmetic product>

A: prepared from ingredients 1 to 9 by mixing with heating.
B: prepared from ingredients 10 to 12 and 14 by mixing with heating.
C: prepared by slowly adding B to A with stirring for emulsification. After cooling, ingredient 13 was added. Thus there was obtained the O/W cream as desired.

The O/W cream obtained as mentioned above was found to spread easily and impart dry feeling without sticky feeling and oily feeling.

### Example 25

### O/W cream

| | Formulation | weight% |
|---|---|---|
| 1. | Polyglyceryl monooleate | 1.0 |
| 2. | Cetyl alcohol | 0.5 |
| 3. | Stearic acid | 1.0 |
| 4. | Glyceryl monostearate | 1.0 |
| 5. | 10 wt% solution of polymer (I-2) in D5 | 1.0 |
| 6. | Macademianut oil | 10.0 |
| 7. | Crosslinked dimethylpolysiloxane (*1) | 0.5 |
| 8. | Acrylates/alkyl acrylate (C10-30) cross-polymer (*2) | 0.2 |
| 9. | Methyl cellulose | 0.1 |
| 10. | Triethanolamine | 0.2 |
| 11. | 1,3-butylene glycol | 7.0 |
| 12. | Antiseptic | q.s. |
| 13. | Perfume | q.s. |
| 14. | Purified water | 77.5 |
| Total | | 100.0 |

| | | |
|---|---|---|
| *1: KSG-16 from Shin-Etsu Chemical Co., Ltd. *2: Pemulen TR-1 from Noveon Inc. | | |

### <Preparation of cosmetic product>

A: prepared from ingredients 1 to 7 by mixing with heating.
B: prepared from ingredients 8 to 12 and 14 by mixing with heating.
C: prepared by slowly adding B to A with stirring for emulsification. After cooling, ingredient 13 was added. Thus there was obtained the O/W cream as desired.

The O/W cream obtained as mentioned above was found to spread easily, impart dry feeling, and keep the skin fresh without sticky feeling and oily feeling.

### Example 26

### Antiperspirant

| | Formulation | weight% |
|---|---|---|
| 1. | Crosslinked polyether-modified silicone (*1) | 7.0 |
| 2. | 10 wt% solution of polymer (I-2) in D5 | 8.0 |
| 3. | Decamethylcyclopentasiloxane | 9.0 |
| 4. | 1,3-butylene glycol | 5.0 |
| 5. | Sodium citrate | 0.2 |
| 6. | Glycine salt of aluminum zirconium tetra-chloride hydrate | 20.0 |
| 7. | Purified water | 50.8 |
| Total | | 100.0 |

| | | |
|---|---|---|
| *1: KSG-210 from Shin-Etsu Chemical Co., Ltd. | | |

### <Preparation of cosmetic product>

A: prepared from ingredients 1 to 3 by uniform mixing.
B: prepared from ingredients 4 to 7 by uniform mixing.
C: prepared by slowly adding B to A with stirring for emulsification. Thus there was obtained the antiperspirant as desired.

The antiperspirant obtained as mentioned above was found to spread easily, last long, and produce antiperspirant effect without whitening the skin.

### Example 27- reference example

### Wrinkle concealer

| | Formulation | weight% |
|---|---|---|
| 1. | Crosslinked polyether-modified silicone (*1) | 5.0 |
| 2. | Crosslinked dimethylpolysiloxane (*2) | 55.0 |
| 3. | 10 wt% solution of polymer (I-2) in D5 | 15.0 |
| 4. | Decamethylcyclopentasiloxane | 8.0 |
| 5. | Hybrid silicone composite powder (*3) | 12.0 |
| 6. | Silicone gum solution (*4) | 5.0 |
| Total | | 100.0 |

| | | |
|---|---|---|
| *1: KSG-210 from Shin-Etsu Chemical Co., Ltd. *2: KSG-15 from Shin-Etsu Chemical Co., Ltd. *3: KSP-101 from Shin-Etsu Chemical Co., Ltd. *4: KF-9028 from Shin-Etsu Chemical Co., Ltd. | | |

### <Preparation of cosmetic product>

A: prepared from ingredients 1 to 6 by uniform mixing.
   Thus there was obtained the wrinkle concealer as desired.

The wrinkle concealer obtained as mentioned above was found to spread easily, impart light feeling, and keep long the concealing effect without sticky feeling and oily feeling.

### Example 28

### Cleansing cream

| | Formulation | weight% |
|---|---|---|
| 1. | Dimethylpolysiloxane (6 cs) | 5.0 |
| 2. | Methylphenylpolysiloxane (*1) | 5.0 |
| 3. | Liquid paraffin | 5.0 |
| 4. | Jojoba oil | 2.0 |
| 5. | 10 wt% solution of polymer (I-2) in D5 | 4.0 |
| 6. | Branched polyether-modified silicone (*2) | 2.0 |
| 7. | Dextrin fatty acid ester | 0.8 |
| 8. | Aluminum monostearate | 0.2 |
| 9. | Aluminum chloride | 1.0 |
| 10. | Glycerin | 10.0 |
| 11. | Antiseptic | q.s. |
| 12. | Perfume | q.s. |
| 13. | Purified water | 65.0 |
| Total | | 100.0 |

| | | |
|---|---|---|
| *1: KF-56 from Shin-Etsu Chemical Co., Ltd. *2: KF-6028 from Shin-Etsu Chemical Co., Ltd. | | |

### <Preparation of cosmetic product>

A: prepared from ingredients 1 to 8 by mixing with heating.
B: prepared from ingredients 9 to 11 and 13 by mixing with heating.
C: prepared by slowly adding B to A with stirring for emulsification. After cooling, ingredient 12 was added. Thus there was obtained the cleansing cream as desired.

The cleansing cream obtained as mentioned above was found to spread easily and impart refresh feeling, moist feeling, and light feeling.

### Example 29

### Transparent cleansing lotion

| | Formulation | weight% |
|---|---|---|
| 1. | Decamethylcyclopentasiloxane | 50.0 |
| 2. | 30 wt% solution of polymer (I-2) in dimethicone (2 cs) | 5.8 |
| 3. | Neopentylglycol dioctanoate | 6.0 |
| 4. | Silica | 0.2 |
| 5. | 1,3-butylene glycol | 5.0 |
| 6. | Glycerin | 6.0 |
| 7. | Polyether-modified silicone (*1) | 5.0 |
| 8. | Polyether-modified silicone (*2) | 3.0 |
| 9. | PEG-60 hydrogenated castor oil | 2.0 |
| 10. | Purified water | 17.0 |
| Total | | 100.0 |

| | | |
|---|---|---|
| *1: KF-6011 from Shin-Etsu Chemical Co., Ltd. *2: KF-6013 from Shin-Etsu Chemical Co., Ltd. | | |

### <Preparation of cosmetic product>

A: prepared from ingredients 1 to 4 by uniform mixing.
B: prepared from ingredients 5 to 10 by uniform mixing.
C: prepared by slowly adding B to A with stirring for emulsification. Thus there was obtained the transparent cleansing lotion as desired.

The transparent cleansing lotion obtained as mentioned above was found to spread easily and impart refresh feeling and moist feeling, and produce good cleansing effect.

### Example 30

### W/O cream

| | Formulation | weight% |
|---|---|---|
| 1. | Crosslinked polyether-modified silicone (*1) | 3.0 |
| 2. | Crosslinked dimethylpolysiloxane (*2) | 20.0 |
| 3. | Crosslinked dimethylpolysiloxane (*3) | 6.0 |
| 4. | Branched polyether-modified silicone (*4) | 1.0 |
| 5. | Decamethylcyclopentasiloxane | 15.0 |
| 6. | Dimethylpolysiloxane (6 cs) | 5.0 |
| 7. | 10 wt% solution of polymer (I-2) in D5 | 5.0 |
| 8. | Dimethyldistearylammoniumhectorite | 2.0 |
| 9. | 1,3-butylene glycol | 10.0 |
| 10. | Sodium chloride | 0.1 |
| 11. | Antiseptic | q.s. |
| 12. | Perfume | q.s. |
| 13. | Purified water | 32.9 |
| Total | | 100.0 |

| | | |
|---|---|---|
| *1: KSG-210 from Shin-Etsu Chemical Co., Ltd. *2: KSG-15 from Shin-Etsu Chemical Co., Ltd. *3: KSG-16 from Shin-Etsu Chemical Co., Ltd. *4: KF-6028P from Shin-Etsu Chemical Co., Ltd. | | |

### <Preparation of cosmetic product>

A: prepared from ingredients 1 to 8 by uniform mixing with heating at 50° C.
B: prepared from ingredients 9 to 11 and 13 by uniform mixing.
C: prepared by slowly adding B to A with stirring for emulsification. Then, ingredient 12 was added. Thus there was obtained the W/O cream as desired.

The W/O cream obtained as mentioned above was found to spread easily and keep the skin moist without sticky feeling and oily feeling.

### Example 31

### W/O blusher

| | Formulation | weight% |
|---|---|---|
| 1. | Acrylsilicone resin solution (*1) | 10.0 |
| 2. | Stearyl-modified acrylsilicone resin (*2) | 2.0 |
| 3. | Branched polyether-modified silicone (*3) | 1.5 |
| 4. | Decamethylcyclopentasiloxane | 15.0 |
| 5. | Glyceryl triisostearate | 3.0 |
| 6. | 10 wt% solution of polymer (I-2) in D5 | 5.0 |
| 7. | Dimethyldistearylammoniumhectorite | 1.5 |
| 8. | Spherical nylon powder | 3.0 |
| 9. | Talc | 4.0 |
| 10. | Blusher pigment (treated with acryl-silicone) (*4) | 20.0 |
| 11. | Alcohol | 5.0 |
| 12. | Perfume | q.s. |
| 13. | Purified water | 30.0 |
| Total | | 100.0 |

| | | |
|---|---|---|
| *1: KP-545 from Shin-Etsu Chemical Co., Ltd. *2: KP-561P from Shin-Etsu Chemical Co., Ltd. *3: KF-6028P from Shin-Etsu Chemical Co., Ltd. *4: treated with KP-574 from Shin-Etsu Chemical Co., Ltd. | | |

### <Preparation of cosmetic product>

A: prepared from ingredients 1 to 7 by mixing with heating.
B: prepared from ingredients 8 to 10 and 12 by uniform mixing, followed by mixing with A.
C: prepared from ingredients 11 and 13 by mixing.
D: prepared by slowly adding B to C with stirring for emulsification. Thus there was obtained the W/O blusher as desired.

The W/O blusher obtained as mentioned above was found to spread easily, last long, and adhere well without sticky feeling and oily feeling.

### Example 32

### W/O liquid foundation

| | Formulation | weight% |
|---|---|---|
| 1. | Crosslinked polyether-modified silicone (*1) | 3.0 |
| 2. | Crosslinked dimethylpolysiloxane (*2) | 5.0 |
| 3. | Branched polyether-modified silicone (*3) | 2.0 |
| 4. | Decamethylcyclopentasiloxane | 20.0 |
| 5. | Cetyl isooctanoate | 5.0 |
| 6. | 10 wt% solution of polymer (I-2) in D5 | 10.0 |
| 7. | Dimethyldistearylammoniumhectorite | 1.2 |
| 8. | Foundation pigment (silicone-treated) (*4) | 14.0 |
| 9. | Acrylsilicone resin solution (*5) | 10.0 |
| 10. | 1,3-butylene glycol | 5.0 |
| 11. | Xanthan gum | 0.1 |
| 12. | Sodium citrate | 0.2 |
| 13. | Sodium chloride | 0.5 |
| 14. | Antiseptic | q.s. |
| 15. | Perfume | q.s. |
| 16. | Purified water | 24.0 |
| Total | | 100.0 |

| | | |
|---|---|---|
| *1: KSG-210 from Shin-Etsu Chemical Co., Ltd. *2: KSG-15 from Shin-Etsu Chemical Co., Ltd. *3: KF-6028P from Shin-Etsu Chemical Co., Ltd. *4: treated with KF-9909 from Shin-Etsu Chemical Co., Ltd. *5: KP-575 from Shin-Etsu Chemical Co., Ltd. | | |

### <Preparation of cosmetic product>

A: prepared from a portion of ingredient 4 and ingredient 9 by mixing. Then ingredient 8 was uniformly dispersed into it.
B: prepared from ingredients 1 to 3, the remainder of ingredient 4, and ingredients 5 to 7 by uniform mixing.
C: prepared from ingredients 10 to 14 and 16 by uniform mixing.
D: prepared by slowly adding B to C with stirring for emulsification. A and ingredient 15 were added. Thus there was obtained the W/O liquid foundation as desired.

The W/O liquid foundation obtained as mentioned above was found to spread easily and last long without sticky feeling and oily feeling and secondary sticking.

### Example 33

### W/O cream

| | Formulation | weight% |
|---|---|---|
| 1. | Crosslinked alkyl·polyether-modified silicone (*1) | 3.0 |
| 2. | Crosslinked alkyl-modified dimethylpolysiloxane (*2) | 4.0 |
| 3. | Alkyl-modified branched polyether-modified silicone (*3) | 1.0 |
| 4. | Meadowfoam oil | 3.5 |
| 5. | Jojoba oil | 2.5 |
| 6. | Macademianut oil | 5.0 |
| 7. | 10 wt% solution of polymer (I-2) in D5 | 7.5 |
| 8. | Hybrid silicone complex powder (*4) | 3.0 |
| 9. | 1,3-butylene glycol | 8.0 |
| 10. | Glycine | 3.0 |
| 11. | Sodium citrate | 0.2 |
| 12. | Sodium chloride | 0.5 |
| 13. | Antiseptic | q.s. |
| 14. | Perfume | q.s. |
| 15. | Purified water | 58.8 |
| Total | | 100.0 |

| | | |
|---|---|---|
| *1: KSG-340 from Shin-Etsu Chemical Co., Ltd. *2: KSG-44 from Shin-Etsu Chemical Co., Ltd. *3: KF-6038 from Shin-Etsu Chemical Co., Ltd. *4: KSP-100 from Shin-Etsu Chemical Co., Ltd. | | |

### <Preparation of cosmetic product>

A: prepared from ingredients 1 to 8 by uniform mixing.
B: prepared from ingredients 9 to 13 and 15 by uniform mixing.
C: prepared by slowly adding B to A with stirring for emulsification. Then, ingredient 14 was added. Thus there was obtained the W/O cream as desired.

The W/O cream obtained as mentioned above was found to keep the skin moist without sticky feeling and oily feeling.

### Example 34- reference example

### Lipstick

| | Formulation | weight% |
|---|---|---|
| 1. | Candelilla wax | 4.0 |
| 2. | Polyethylene | 2.0 |
| 3. | Microcrystalline wax | 3.0 |
| 4. | Seresin | 7.0 |
| 5. | Stearyl-modified acrylsilicone resin (*1) | 15.0 |
| 6. | Diphenyldimethicone (*2) | 20.0 |
| 7. | 10 wt% solution of polymer (I-2) in D5 | 10.0 |
| 8. | Alkyl-modified branched polyglycerin-modified silicone(*3) | 3.0 |
| 9. | Macademianut oil | 20.0 |
| 10. | Hydrogenated polyisobutene | 10.0 |
| 11. | Isotridecyl isononanonate | 6.0 |
| 12. | Perfume | q.s. |
| 13. | Lipstick pigment | q.s. |
| 14. | Mica | q.s. |
| Total | | 100.0 |

| | | |
|---|---|---|
| *1: KP-561P from Shin-Etsu Chemical Co., Ltd. *2: KF-54 from Shin-Etsu Chemical Co., Ltd. *3: KF-6105 from Shin-Etsu Chemical Co., Ltd. | | |

### <Preparation of cosmetic product>

A: prepared from ingredients 1 to 12 by uniform mixing with heating.
B: prepared by adding ingredients 13 and 14 to A, followed by uniform mixing with heating. The resulting mixture was filled into air-tight containers. Thus, there was obtained the lipstick as desired.

The lipstick obtained as mentioned above was found to last long without blotting, sticky feeling, and oily feeling.

### Example 35

### Cuticle coat

| | Formulation | weight% |
|---|---|---|
| 1. | Polyether-modified silicone (*1) | 3.0 |
| 2. | Polyether-modified silicone (*2) | 2.0 |
| 3. | PEG-40 hydrogenated hardened castor oil | 1.0 |
| 4. | 10 wt% solution of polymer (I-2) in D5 | 3.0 |
| 5. | Silicone gum solution (*3) | 40.0 |
| 6. | Decamethylcyclopentasiloxane | 40.0 |
| 7. | Alcohol | 5.0 |
| 8. | Antiseptic | q.s. |
| 9. | Perfume | q.s. |
| 10. | Purified water | 6.0 |
| Total | | 100.0 |

| | | |
|---|---|---|
| *1: KF-6011 from Shin-Etsu Chemical Co., Ltd. *2: KF-6013 from Shin-Etsu Chemical Co., Ltd. *3: KF-9028 from Shin-Etsu Chemical Co., Ltd. | | |

### <Preparation of cosmetic product>

A: prepared from ingredients 1 to 3 and 7 to 10 by uniform mixing.
B: prepared from ingredients 4 to 6 by uniform mixing.
C: prepared by adding B to A with stirring for emulsification. Thus there was obtained the cuticle coat as desired.

The cuticle coat obtained as mentioned above was found to spread easily and make the hair lustrous and smooth while preventing the hair from becoming dry and loose.

### Example 36

### Hair treatment

| | Formulation | weight% |
|---|---|---|
| 1. | Silicone gum solution (*1) | 5.0 |
| 2. | Diphenyldimethicone (*2) | 4.0 |
| 3. | 10 wt% solution of polymer (I-2) in D5 | 1.0 |
| 4. | Cetyl octanoate | 1.0 |
| 5. | Cetyl alcohol | 0.5 |
| 6. | Polyether-modified silicone (*3) | 1.0 |
| 7. | PEG-60 hydrogenated hardened castor oil | 1.0 |
| 8. | Glyceryl monostearate | 0.5 |
| 9. | Carboxyvinylpolymer (1 wt% aqueous solution) | 25.0 |
| 10. | Xanthan gum (1 wt% aqueous solution) | 7.0 |
| 11. | 1,3-butylene glycol | 5.0 |
| 12. | Alcohol | 7.0 |
| 13. | Antiseptic | q.s. |
| 14. | Perfume | q.s. |
| 15. | Purified water | 42.0 |
| | Total | 100.0 |

| | | |
|---|---|---|
| *1: MK-15H from Shin-Etsu Chemical Co., Ltd. *2: KF-54 from Shin-Etsu Chemical Co., Ltd. *3: KF-6013 from Shin-Etsu Chemical Co., Ltd. | | |

### <Preparation of cosmetic product>

A: prepared from ingredients 1 to 8 by dissolution with heating.
B: prepared from ingredients 11 to 15 by dissolution with heating.
C: prepared by adding B to A with stirring for emulsification. Ingredients 8 and 9 were added. Thus there was obtained the hair treatment as desired.

The hair treatment obtained as mentioned above was found to spread easily and make the hair lustrous and smooth.

### Example 37

### Nail enamel

| | Formulation | weight% |
|---|---|---|
| 1. | 30 wt% solution of polymer (I-1) in M3T | 45.0 |
| 2. | Methyltrimethicone (*1) | 5.0 |
| 3. | Nitrocellulose | 3.0 |
| 4. | Camphor | 0.5 |
| 5. | Acetyltributyl citrate | 1.0 |
| 6. | Dimethyldistearylammoniumhectorite | 0.5 |
| 7. | Butyl acetate | 30.0 |
| 8. | Ethyl acetate | 10.0 |
| 9. | Isopropyl alcohol | 5.0 |
| 10. | Colored pigment | q.s. |
| | Total | 100.0 |

| | | |
|---|---|---|
| *1: TMF-1.5 from Shin-Etsu Chemical Co., Ltd. | | |

### <Preparation of cosmetic product>

A: prepared from ingredients 7 to 9 by mixing. Ingredients 4 to 6 were added, followed by uniform mixing.
B: prepared by adding ingredients 1 to 3 to A, followed by mixing.
C: prepared by adding ingredient 10 to B. Thus there was obtained the nail enamel as desired.

The nail enamel obtained as mentioned above was found to spread easily, impart luster to the nail, and last long.

### Example 38

### Nail enamel overcoat

| | Formulation | weight% |
|---|---|---|
| 1. | 30 wt% solution of polymer (I-2) in isododecane | 6.0 |
| 2. | Nitrocellulose | 17.0 |
| 3. | Alkyd resin | 4.0 |
| 4. | Acetyltriethyl citrate | 5.0 |
| 5. | Butyl acetate | 29.0 |
| 6. | Ethyl acetate | 25.0 |
| 7. | Isopropyl alcohol | 3.0 |
| 8. | n-butyl alcohol | 1.0 |
| 9. | Toluene | 10.0 |
| | Total | 100.0 |

### <Preparation of cosmetic product>

A: prepared from ingredients 5 to 9 by mixing. Ingredient 4 was added, followed by uniform mixing.
B: prepared by adding ingredients 1 to 3 to A, followed by mixing. Thus there was obtained the nail enamel overcoat as desired.

The nail enamel overcoat obtained as mentioned above was found to spread easily, make the enamel more lustrous, and last long.

## Claims

1. A cosmetic product comprising
(A) polymer having the repeating unit represented by the following formula (1) or (2): 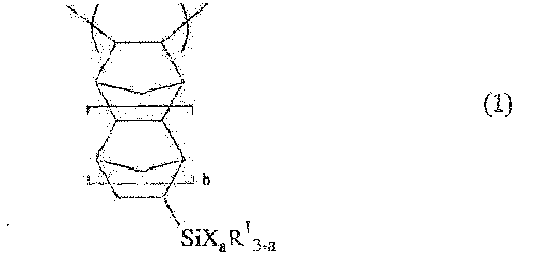 wherein R¹ is independently alkyl group having 1 to 12 carbon atoms, X is a group represented by the following formula (i), a is an integer of 1 to 3, and b is an integer of 0 to 2, 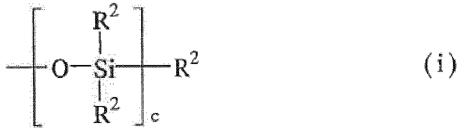 wherein R² is independently alkyl group having 1 to 12 carbon atoms and c is an integer of 1 to 5, 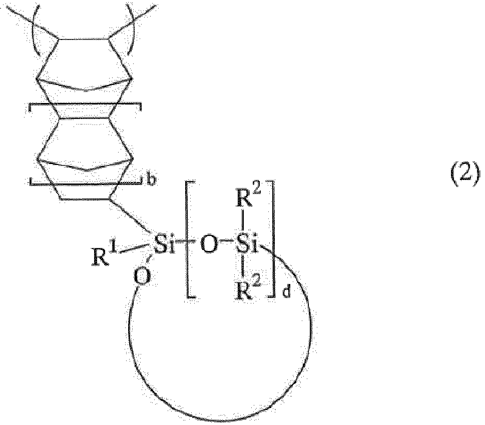 wherein R¹, R², and b are defined as above, and d is an integer of 2 to 5;
and additionally
(C) compound which contains alcoholic hydroxyl group in its molecular structure,
wherein the cosmetic product is
a powder cosmetic product containing from 0.1 to 10 wt% of said polymer, or
a liquid cosmetic product containing from 0.1 to 7.5 wt% of said polymer
and is selected from toilet water, milky lotion, cream, cleansing, pack, massage oil, facial lotion, facial oil, cleansing agent, deodorant, hand cream, lip cream, skin care product to hide wrinkles, make-up base, concealer, white make-up powder, powder foundation, liquid foundation, cream foundation, oil foundation, blush-on product, eye shadow, mascara, eye liner, eye brow, lipstick, manicure products, hair cosmetics, antiperspirants and ultraviolet protective cosmetic preparations such as sunscreen oil, sunscreen milky emulsion, and sunscreen cream.

2. A cosmetic product according to claim 1 in which said repeating units of formula (1) or (2) constitute from 40 to 95% of all the repeating units in said polymer.

3. A cosmetic product according to claim 1 or 2 in which in formula (1) R¹ and R² are methyl groups, or in formula (2) R² is methyl group and d is 2 or 3.

4. A cosmetic product according to any one of the preceding claims wherein said polymer further has the repeating unit represented by the following formula (3): 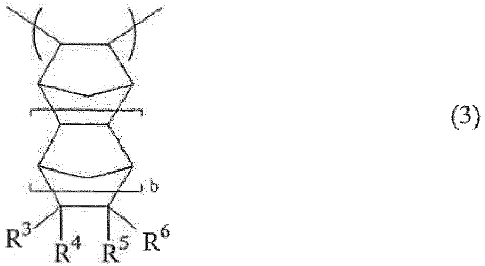 wherein b is an integer of 0 to 2, and
R³ to R⁶ are independently selected from the group consisting of
hydrogen atoms, halogen atoms,
substituents selected from C1-10 alkyl groups, alkenyl groups, cycloalkyl groups, aryl groups, alkoxy groups, aryloxy groups and halogenated hydrocarbon groups,
polar groups selected from oxetanyl groups, alkoxycarbonyl groups, polyoxyalkylene groups and polyglyceryl groups, and
alkoxysilyl groups,
provided that any two of R³ to R⁶ may form, in conjunction with carbon atoms to which they join, an alicyclic structure, aromatic ring structure, carbonimide group, or acid anhydride.

5. A cosmetic product according to claim 4 in which repeating units of formula (3) are 5 to 60% of all the repeating units in the polymer.

6. A cosmetic product according to any one of the preceding claims wherein said polymer has a number-average molecular weight (Mn) of 10,000 to 2,000,000, as determined by GPC with reference to polystyrene standards.

7. A cosmetic product according to claim 6 in which said number average molecular weight (Mn) is from 100,000 to 800,000.

8. A cosmetic product according to any one of the preceding claims further comprising (B) oil which is a volatile silicone oil.

9. A cosmetic product according to claim 8, wherein said volatile silicone oil is selected from octamethyltrisiloxane, decamethyltetrasiloxane, octamethylcyclotetrasiloxane, decamethylcyclopentasiloxane, tristrimethylsiloxymethylsilane, tetrakistrimethylsiloxysilane and dodecamethylpentasiloxane.

10. A cosmetic product according to any one of claims 1 to 7 further comprising isododecane.

11. A cosmetic product according to any one of the preceding claims wherein (C) compound is selected from the group consisting of ethanol, isopropanol, butanol, sorbitol, maltose, cholesterol, sitosterol, phytosterol, lanosterol, butylene glycol, propylene glycol, dibutylene glycol, and pentylene glycol.

12. A cosmetic product according to any one of the preceding claims which is in the form of powder, oil, oil-in-water emulsion, water-in-oil emulsion, non-aqueous emulsion, W/O/W multiemulsion or O/W/O multiemulsion.

13. Use of a product according to any one of claims 1 to 12 as a cosmetic product.

## Patentansprüche

1. Kosmetikprodukt, umfassend
(A) ein Polymer, das die Grundeinheit der folgenden Formel (1) oder (2) aufweist: 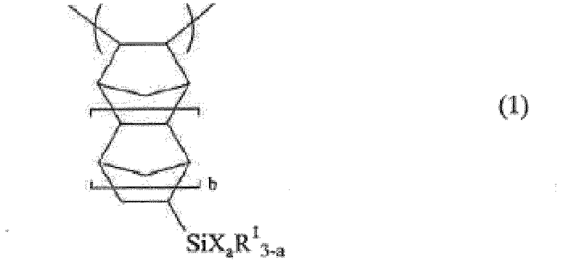 worin R¹ unabhängig eine Alkylgruppe mit 1 bis 12 Kohlenstoffatomen ist, X eine Gruppe der folgenden Formel (i) ist, a eine ganze Zahl von 1 bis 3 ist und b eine ganze Zahl von 0 bis 2 ist, 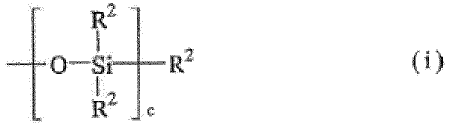 worin R² unabhängig eine Alkylgruppe mit 1 bis 12 Kohlenstoffatomen und c eine ganze Zahl von 1 bis 5 ist, 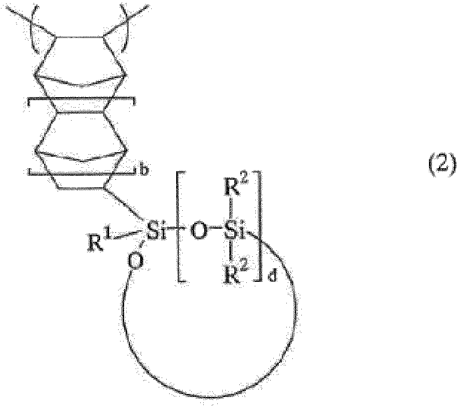 worin R¹, R² und b wie oben definiert sind und d eine ganze Zahl von 2 bis 5 ist; und zusätzlich
(C) eine Verbindung, die eine alkoholische Hydroxylgruppe in ihrer Molekülstruktur enthält,
wobei das Kosmetikprodukt
ein pulverförmiges Kosmetikprodukt ist, das 0,1 bis 10 Gew.-% des Polymers enthält, oder
ein flüssiges Kosmetikprodukt ist, das 0,1 bis 7,5 Gew.-% des Polymers enthält,
und aus einem Duftwasser, einer Milchlotion, einer Creme, einer Reinigungslotion, einer Packung, einem Massageöl, einer Gesichtslotion, einem Gesichtsöl, einem Reinigungsmittel, einem Deodorant, einer Handcreme, einer Lippencreme, einem Hautpflegeprodukt zum Verbergen von Falten, einer Make-up-Basis, einem Concealer, einem weißen Make-up-Puder, einer Pulverbasis, einer Flüssigbasis, einer Cremebasis, einer Ölbasis, einem Rouge-Produkt, einem Lidschatten, einer Mascara, einem Eyeliner, einem Augenbrauenstift, einem Lippenstift, Maniküreprodukten Haarkosmetika, Antitranspirantien und UV-Schutzkosmetikpräparaten, wie z.B. Sonnenschutzöl, Sonnenschutzmilchemulsion und Sonnenschutzcreme, ausgewählt ist.

2. Kosmetikprodukt nach Anspruch 1, bei dem die Grundeinheiten der Formel (1) oder (2) 40 bis 95 % aller Grundeinheiten in dem Polymer ausmachen.

3. Kosmetikprodukt nach Anspruch 1 oder 2, bei dem R¹ und R² in Formel (1) Methylgruppen sind oder R² in Formel (2) eine Methylgruppe ist und d = 2 oder 3 ist.

4. Kosmetikprodukt nach einem der vorangegangenen Ansprüche, wobei das Polymer zudem eine Grundeinheit der folgenden Formel (3) aufweist: 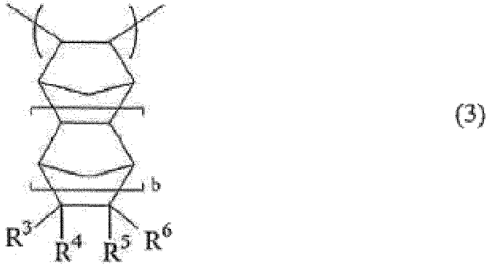 wobei b eine ganze Zahl von 0 bis 2 ist und
R³ bis R⁶ unabhängig aus der aus Folgendem bestehenden Gruppe ausgewählt sind:
Wasserstoffatomen, Halogenatomen,
Substituenten, die aus C₁-C₁₀-Alkylgruppen, Alkenylgruppen, Cycloalkylgruppen, Arylgruppen, Alkoxygruppen, Aryloxygruppen und halogenierten Kohlenwasserstoffgruppen ausgewählt sind,
polaren Gruppen, die aus Oxetanylgruppen, Alkoxycarbonylgruppen, Polyoxyalkylengruppen und Polyglycerylgruppen ausgewählt sind, und
Alkoxysilylgruppen,
mit der Maßgabe, dass gegebenenfalls beliebige zwei von R³ bis R⁶ zusammen mit den Kohlenstoffatomen, an die sie gebunden sind, eine alizyklische Struktur, eine aromatische Ringstruktur, eine Carbonimidgruppe oder ein Säureanhydrid bilden.

5. Kosmetikprodukt nach Anspruch 4, bei dem Grundeinheiten der Formel (3) 5 bis 60 % aller Grundeinheiten im Polymer ausmachen.

6. Kosmetikprodukt nach einem der vorangegangenen Ansprüche, wobei das Polymer ein zahlenmittleres Molekulargewicht (Mn) von 10.000 bis 2.000.000, gemessen mittels GPC in Bezug auf Polystyrol-Standards, aufweist.

7. Kosmetikprodukt nach Anspruch 6, bei dem das zahlenmittlere Molekulargewicht (Mn) 100.000 bis 800.000 beträgt.

8. Kosmetikprodukt nach einem der vorangegangenen Ansprüche, das weiters (B) ein Öl umfasst, das ein flüchtiges Silikonöl ist.

9. Kosmetikprodukt nach Anspruch 8, wobei das flüchtige Silikonöl aus Octamethyltrisiloxan, Decamethyltetrasiloxan, Octamethylcyclotetrasiloxan, Decamethylcyclopentasiloxan, Tristrimethylsiloxymethylsilan, Tetrakistrimethylsiloxysilan und Dodecamethylpentasiloxan ausgewählt ist.

10. Kosmetikprodukt nach einem der Ansprüche 1 bis 7, das weiters Isododecan umfasst.

11. Kosmetikprodukt nach einem der vorangegangenen Ansprüche, wobei Verbindung (C) aus der aus Ethanol, Isopropanol, Butanol, Sorbit, Maltose, Cholesterin, Sitosterin, Phytosterin, Lanosterin, Butylenglykol, Propylenglykol, Dibutylenglykol und Pentylenglykol bestehenden Gruppe ausgewählt ist.

12. Kosmetikprodukt nach einem der vorangegangenen Ansprüche, das in Form eines Pulvers, eines Öls, einer Öl-in-Wasser-Emulsion, einer Wasser-in-Öl-Emulsion, einer nichtwässrigen Emulsion, einer W/O/W-Multiemulsion oder O/W/O-Multiemulsion vorliegt.

13. Verwendung eines Produkts nach einem der Ansprüche 1 bis 12 als Kosmetikprodukt.

## Revendications

1. Produit cosmétique comprenant
(A) un polymère ayant un motif répétitif représenté par la formule (1) ou (2) qui suit : 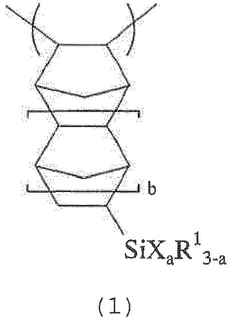 dans laquelle R¹ est indépendamment un groupe alkyle ayant 1 à 12 atomes de carbone, X est un groupe représenté par la formule (i) suivante, a est un entier de 1 à 3, et b est un entier de 0 à 2, 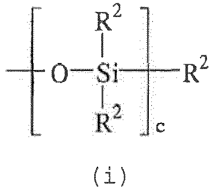 dans laquelle R² est indépendamment un groupe alkyle ayant 1 à 12 atomes de carbone et c est un entier de 1 à 5, 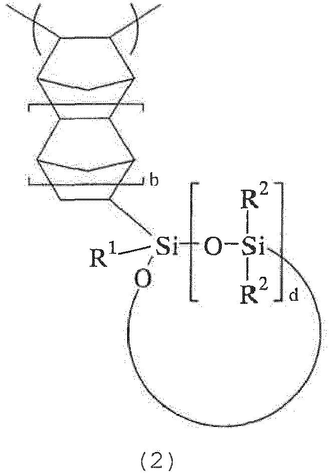 dans laquelle R¹, R² et b sont tels que définis ci-dessus, et d est un entier de 2 à 5 ;
et de plus
(C) un composé qui contient un groupe hydroxyle alcoolique dans sa structure moléculaire,
lequel produit cosmétique est
un produit cosmétique en poudre contenant de 0,1 à 10 % en poids dudit polymère, ou
un produit cosmétique liquide contenant de 0,1 à 7,5 % en poids dudit polymère,
et est choisi parmi une eau de toilette, un lait, une crème, un nettoyant, une compresse, une huile de massage, une lotion pour le visage, une huile pour le visage, un agent nettoyant, un déodorant, une crème pour les mains, une crème pour les lèvres, un produit de soin de la peau pour masquer les rides, une base de maquillage, un correcteur de teint, une poudre de maquillage blanche, un fond de teint en poudre, un fond de teint liquide, un fond de teint en crème, un fond de teint en huile, un produit de fard à joues, une ombre à paupières, un mascara, un eyeliner, un produit pour sourcils, un rouge à lèvres, un produit de manucure, un cosmétique capillaire, un antiperspirant et une préparation cosmétique protégeant contre les ultraviolets telle qu'une huile écran solaire, une émulsion laiteuse écran solaire, et une crème écran solaire.

2. Produit cosmétique selon la revendication 1, dans lequel lesdits motifs répétitifs de formule (1) ou (2) constituent de 40 à 95 % de tous les motifs répétitifs dans ledit polymère.

3. Produit cosmétique selon la revendication 1 ou 2, dans lequel, dans la formule (1), R¹ et R² sont des groupes méthyle, ou, dans la formule (2), R² est un groupe méthyle et d vaut 2 ou 3.

4. Produit cosmétique selon l'une quelconque des revendications précédentes, dans lequel ledit polymère a en outre le motif répétitif représenté par la formule (3) suivante 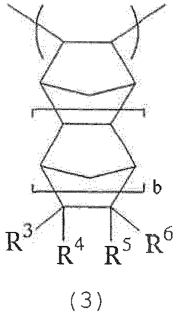 dans laquelle b est un entier de 0 à 2, et
R³ à R⁶ sont indépendamment choisis dans l'ensemble constitué par
les atomes d'hydrogène, les atomes d'halogène,
les substituants choisis parmi les groupes alkyle en C₁ à C₁₀, les groupes alcényle, les groupes cycloalkyle, les groupes aryle, les groupes alcoxy, les groupes aryloxy et les groupes hydrocarbonés halogénés,
les groupes polaires choisis parmi les groupes oxétanyle, les groupes alcoxycarbonyle, les groupes polyoxyalkylène et les groupes polyglycéryle, et
les groupes alcoxysilyle,
sous réserve que deux quelconques parmi R³ à R⁶ puissent former, conjointement avec les atomes de carbone auxquels ils sont joints, une structure alicyclique, une structure cyclique aromatique, un groupe carbonimide, ou un anhydride d'acide.

5. Produit cosmétique selon la revendication 4, dans lequel les motifs répétitifs de formule (3) représentent 5 à 60 % de tous les motifs répétitifs dans le polymère.

6. Produit cosmétique selon l'une quelconque des revendications précédentes, dans lequel ledit polymère a une masse moléculaire moyenne en nombre (Mn) de 10 000 à 2 000 000, telle que déterminée par GPC en référence à des étalons de polystyrène.

7. Produit cosmétique selon la revendication 6, dans lequel ladite masse moléculaire moyenne en nombre (Mn) est de 100 000 à 800 000.

8. Produit cosmétique selon l'une quelconque des revendications précédentes, comprenant en outre (B) une huile qui est une huile siliconée volatile.

9. Produit cosmétique selon la revendication 8, dans lequel ladite huile siliconée volatile est choisie parmi l'octaméthyltrisiloxane, le décaméthyltétrasiloxane, l'octaméthylcyclotétrasiloxane, le décaméthylcyclopentasiloxane, le tristriméthylsiloxyméthylsilane, le tétrakistriméthylsiloxysilane et le dodécaméthylpentasiloxane.

10. Produit cosmétique selon l'une quelconque des revendications 1 à 7, comprenant en outre de l'isododécane.

11. Produit cosmétique selon l'une quelconque des revendications précédentes, dans lequel le composé (C) est choisi dans l'ensemble constitué par l'éthanol, l'isopropanol, le butanol, le sorbitol, le maltose, le cholestérol, le sitostérol, le phytostérol, le lanostérol, le butylèneglycol, le propylèneglycol, le dibutylèneglycol, et le pentylèneglycol.

12. Produit cosmétique selon l'une quelconque des revendications précédentes, qui est sous la forme d'une poudre, d'une huile, d'une émulsion huile dans l'eau, d'une émulsion eau dans l'huile, d'une émulsion non aqueuse, d'une multi-émulsion eau dans l'huile dans l'eau, ou d'une multi-émulsion huile dans l'eau dans l'huile.

13. Utilisation d'un produit selon l'une quelconque des revendications 1 à 12 en tant que produit cosmétique.
